(19) Europäisches Patentamt
European Patent Office
Office européen des brevets

(11) **EP 4 456 809 B1**

(12) **EUROPEAN PATENT SPECIFICATION**

(45) Date of publication and mention
of the grant of the patent:
**29.04.2026 Bulletin 2026/18**

(21) Application number: **22835084.9**

(22) Date of filing: **23.12.2022**

(51) International Patent Classification (IPC):
**A61B 18/22** (2006.01)

(52) Cooperative Patent Classification (CPC):
**A61B 18/22; A61B 5/0075; A61B 5/0086;
A61B 5/065; A61B 5/4836; A61B 5/4848;
A61B 5/6852;** A61B 5/0066; A61B 5/0077;
A61B 18/24; A61B 2017/00061; A61B 2017/00482;
A61B 2018/00517; A61B 2018/00577;
A61B 2018/00642; (Cont.)

(86) International application number:
**PCT/EP2022/087693**

(87) International publication number:
**WO 2023/126337 (06.07.2023 Gazette 2023/27)**

(54) **DISPOSABLE FIBER-OPTIC DEVICE**

FASEROPTISCHE EINWEGVORRICHTUNG

DISPOSITIF JETABLE À FIBRES OPTIQUES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **30.12.2021 EP 21218293**

(43) Date of publication of application:
**06.11.2024 Bulletin 2024/45**

(73) Proprietor: **Optheras A/S**
**3460 Birkerød (DK)**

(72) Inventors:
• **HERMANN, Gregers Gautier**
**3540 Lynge (DK)**

• **ALKESKJOLD, Thomas**
**3540 Lynge (DK)**
• **MOGENSEN, Karin**
**3540 Lynge (DK)**
• **PETERSEN, Søren**
**3540 Lynge (DK)**

(74) Representative: **Guardian**
**IP Consulting I/S**
**Diplomvej, Building 381**
**2800 Kgs. Lyngby (DK)**

(56) References cited:
**WO-A1-2020/112864    US-A1- 2007 282 403
US-B1- 9 494 738**

(52) Cooperative Patent Classification (CPC): (Cont.)
A61B 2018/00708; A61B 2018/00785;
A61B 2018/00791; A61B 2018/00982;
A61B 2018/2244; A61B 2562/0233; G02B 6/02042;
G02B 6/262; G02B 6/3807

**Description**

TECHNICAL FIELD

The present disclosure relates to a disposable fiber-optic device and to a fiber-optic medical treatment apparatus comprising such a disposable fiber device.

BACKGROUND

Many medical conditions can be treated by applying laser light to tissue to be treated. Often, such treatment is performed by directing treatment laser radiation via an optical fiber towards the tissue to be treated. In this context, documents US 2007/282403 A1 and WO 2020/112864 A1 are referred to.

Moreover, in some applications, in addition to directing the treatment laser radiation to the tissue to be treated, it is desirable to also feed other radiation towards the tissue to be treated. For example, when treatment is done in non-contact mode, the distance between the fiber tip and the tissue to be treated has a big impact on the treatment speed and risk, e.g. because accurate manual maneuvering of the fiber can be difficult, in particular if the patient moves or if the tissue moves due to internal movements of the patient's body. It is therefore desirable to measure the distance between the fiber tip and the tissue to be treated.

US 2010/0228119 discloses a system for measuring relative motion during surgical procedure. This prior art system is for use with a cannula that is insertable into human and animal tissue. The system includes a probe having a probe tip that is insertable into the cannula, and means for determining relative motion between the cannula and the probe tip.

In many procedures, an optical fiber is advanced through the working channel of a flexible endoscope. To this end, endoscopes tailored for different types of medical procedures exist. For example, a cystoscope is an endoscope that is configured for performing a cystoscopy where the cystoscope is advanced through the urethra into the urinary bladder. Among other procedures, cystoscopes are used for laser treatment of cancer of the urinary bladder.

Cystoscopes and other types of endoscopes are limited in diameter and, in many procedures, it is desirable that the endoscope is highly bendable. For example, the working channel of cystoscopes typically has a diameter of as little as 1.8 mm. During use, the cystoscope will often be bent to a bending radius of 10 mm radius or to an even smaller bending radius, which may cause the effective diameter of the working channel to be further reduced. Accordingly, it is desirable that the optical fiber used for such procedures is also very thin and highly bendable.

Moreover, the above challenges are further amplified if, in addition to the treatment laser radiation, additional sensor radiation is to be fed towards the tissue to be treated, e.g. for the purpose of distance measurements. It is thus desirable to feed different types of radiation through the working channel of the endoscope via a fiber-optic device, without damaging the fiber-optic device and without unduly degrading the different types of radiation. This may be challenging, as treatment and sensor radiation may impose different and sometimes even opposing requirements or limitations on the fiber-optic device.

Accordingly, it is desirable to provide a fiber-optic device with an optical fiber that can be advanced through even a narrow working channel of an endoscope, such as a cystoscope.

It is further desirable to provide a fiber-optic device with an optical fiber that is highly bendable.

As the fiber-optic device is disposable, e.g. is discarded or recycled after a single or at least after few uses, it is further desirable to keep manufacturing costs of the fiber-optic device low.

It is further desirable to provide a fiber-optic device that can direct different types of radiation towards a target region.

It is further desirable to provide a fiber-optic medical treatment apparatus that allows accurate and efficient monitoring of the distance between the fiber tip and the tissue to be treated and/or accurate and efficient monitoring of the status or progress of the treatment.

SUMMARY

The invention is defined in the appended claims.

Aspects of the present disclosure address at least some of the above matters, and others.

In particular, the present disclosure provides embodiments of a disposable fiber-optic device. The disposable fiber-optic device comprises an at least double-clad optical fiber having a proximal end and a distal end, the distal end being configured to be advanced through a working channel of an endoscope, wherein the at least double-clad optical fiber has at least one core and at least two claddings. The disposable fiber-optic device further comprises a connector module for optically connecting the proximal end of the at least double-clad optical fiber to a medical treatment device. The connector module comprises:

- a first optical fiber,
- a second optical fiber,
- a first optical connector for detachably and optically connecting the first optical fiber to the medical treatment device,
- a second optical connector for detachably and optically connecting the second optical fiber to the medical treatment device, and
- an optical combiner module configured to couple radiation between the first optical fiber and the at least one core of the at least double-clad optical fiber and to couple radiation between the second optical fiber and at least one cladding of the at least two claddings of the at least double-clad optical fiber.

Accordingly, the disposable fiber-optic device allows different types of radiation to be fed through a single at least double-clad optical fiber, the optical fiber may thus be kept thin and highly bendable.

Moreover, as radiation coupled into the core and the cladding of the at least double-clad optical fiber, respectively, via different optical connectors, different types of radiation can efficiently be fed through the single optical fiber, while being able to adapt the different optical connectors to the respective types of radiation, e.g. to high-power treatment laser radiation and low-power sensor radiation that is sensitive to signal disturbances, respectively.

According to another aspect, disclosed herein are embodiments of a fiber-optic medical treatment apparatus comprising a medical treatment device and a disposable fiber-optic device as disclosed above and in the following. The disposable fiber-optic device is configured to be detachably and optically coupled to the medical treatment device.

Those skilled in the art will recognize still other aspects of the present application upon reading and understanding the attached description.

BRIEF DESCRIPTION OF THE DRAWINGS

The above and other aspects are illustrated by way of example and not limited by the figures of the accompanying drawings, in which like references indicate similar elements and in which:

FIG. 1 diagrammatically illustrates an example of a fiber-optic medical treatment apparatus in accordance with embodiments disclosed herein;
FIG. 2 diagrammatically illustrates an example of a disposable fiber-optic device with its optical fiber having been inserted into and advanced through the working channel of an endoscope in accordance with embodiments disclosed herein;
FIG. 3 diagrammatically illustrates an example of a disposable fiber-optic device in accordance with embodiments disclosed herein;
FIG. 4 diagrammatically illustrates an example of an optical combiner module of a connector module of a disposable fiber-optic device in accordance with embodiments disclosed herein;
FIG. 5 diagrammatically illustrates another example of an optical combiner module of a connector module of a disposable fiber-optic device in accordance with embodiments disclosed herein, wherein the at least double-clad optical fiber includes a tapered region;
FIG. 6 diagrammatically illustrates a cross-sectional view of an example of a multi-core double-clad optical fiber for use in a disposable fiber-optic device in accordance with embodiments disclosed herein;
FIG. 7 diagrammatically illustrates operation of the tapered region of the optical combiner module of FIG. 5.
FIGs. 8A-F diagrammatically illustrate cross-sectional views of further examples of an at least double-clad optical fiber for use in a disposable fiber-optic device in accordance with embodiments disclosed herein;
FIGs. 9A-C diagrammatically illustrate longitudinal sectional views of examples of fiber tip designs of an at least double-clad optical fiber for use in a disposable fiber-optic device in accordance with embodiments disclosed herein;
FIG. 10 diagrammatically illustrates a more detailed example of a fiber-optic medical treatment apparatus in accordance with embodiments disclosed herein;
FIG. 11 diagrammatically illustrates an example of an optical combiner module for combining multiple optical paths to be fed through an at least double-clad optical fiber;
FIGs. 12 and 13 diagrammatically illustrate further examples of a disposable fiber-optic device in accordance with embodiments disclosed herein
FIGs. 14A and 14B schematically illustrate an example of a first optical connector of an embodiment of a disposable fiber-optic device as disclosed herein;
FIGs. 15A and 15B schematically illustrate an example of a second optical connector of an embodiment of a disposable fiber-optic device as disclosed herein.

DETAILED DESCRIPTION

The following describes embodiments of a disposable fiber-optic device that mitigate one or more of the above-noted and/or other shortcomings of existing devices or that can at least serve as an alternative to existing devices. The following further describes embodiments of a fiber-optic medical treatment apparatus.

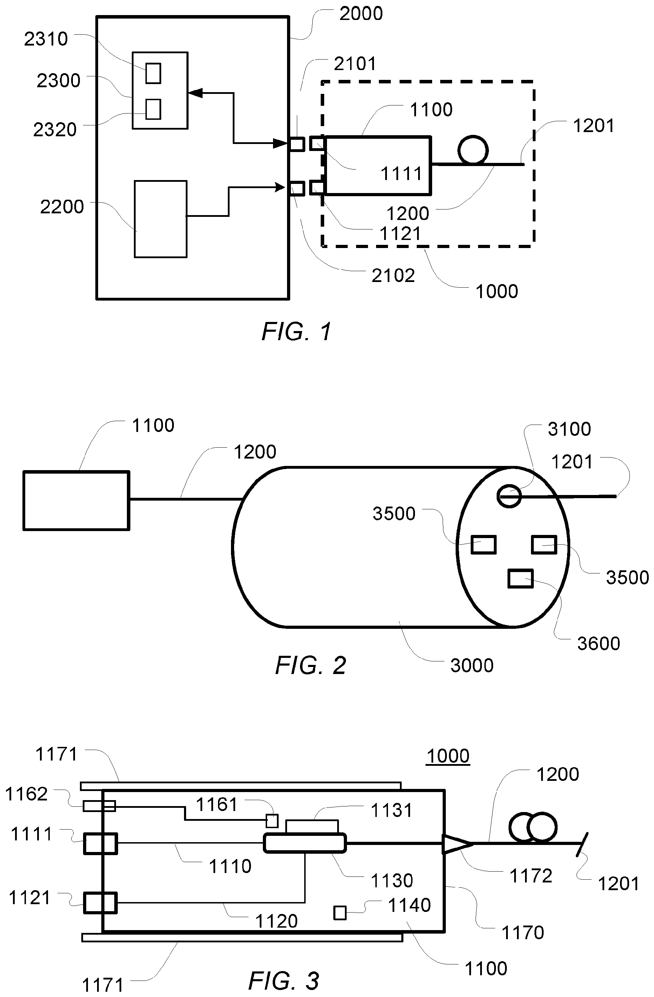

FIG. 1 diagrammatically illustrates an example of a fiber-optic medical treatment apparatus in accordance with embodiments disclosed herein.

The fiber-optic medical treatment apparatus comprises a medical treatment device 2000 and a disposable fiber-optic device 1000. The disposable fiber-optic device 1000 is configured to be detachably and optically coupled to the medical treatment device.

To this end, the disposable fiber-optic device 1000 comprises an at least double-clad optical fiber 1200 and a connector module 1100.

The optical fiber 1200 is at least double-clad, i.e. it may be double-clad or it may be multi-clad with more than two claddings, e.g. triple-clad. Generally, a double-clad optical fiber comprises three layers of optical material. The inner-most layer is called the core. It is surrounded by an inner cladding, which is surrounded by an outer cladding. The three layers are typically made of materials with different refractive indices. The distal end 1201 of the at least double-clad optical fiber 1200 is configured to be advanced through a working channel of an endoscope, such as a cystoscope. Preferably, the core is a single-mode core, single-mode at least at one or more target wavelengths or range of target wavelengths. Optionally, the at least double-clad optical fiber 1200 includes two or more cores, preferably single-mode cores, e.g. a few-mode or single-mode central core and one or more few-mode or single-mode side cores. Optionally, the at least double-clad optical fiber has a medical-grade buffer coating, such as blue EFTE, Teflon, Nylon etc. The optical fiber may have a round cladding or a hexagonal or octagonal cladding for mode scrambling. The inner cladding may be a multi-core cladding.

The at least double-clad optical fiber 1200 may have a diameter suitable for being inserted into the working channel of an endoscope, i.e. the choice of diameter may depend on the type of endoscope. Suitable diameters may be between 0.1 mm and 1 mm, such as between 0.1 mm and 0.4 mm, preferably between 0.1 mm and 0.3 mm, such as between 0.1 mm and 0.25 mm. Optical fibers having a diameter of 1 mm or less, preferably 0.4 mm or less, such as 0.3 mm or less are sufficiently thin to allow bending with relatively little induced tensile and compressive stress on the fiber edges. The length of the at least double-clad optical fiber may depend on the type of endoscope and/or the type of medical procedure. Suitable lengths may be between 2 m and 4 m.

Generally, at least in some embodiments, the at least double-clad optical fiber which emits the radiation towards the tissue to be treated may have a fiber diameter between 100 $\mu$m and 1000 $\mu$m, such as between 100 $\mu$m and 300 $\mu$m, such as between 150 $\mu$m and 200 $\mu$m. In some embodiments the numerical aperture of the multi-mode cladding, which conveys the treatment laser radiation has a numerical aperture of between 0.17 and 0.50, such as between 0.22 and 0.45. Here, the numerical aperture of the inner cladding may be defined as the square root of the difference between the refractive index squared of the inner cladding and the refractive index squared of the outer cladding, which surrounds the inner cladding.

The distal end 1201 of the at least double-clad optical fiber 1200 may be angled, preferably with rounded edges for easy insertion into the working channel of the endoscope and reduced risk of edges of the optical fiber breaking of during insertion.

The connector module 1100 is configured for optically connecting the proximal end of the at least double-clad optical fiber 1200 to the medical treatment device 2000, in particular for separately coupling a core and a cladding, respective, of the at least double-clad optical fiber to the medical treatment device.

To this end, the connector module has a first optical connector 1111 and a second optical connector 1121. The first optical connector 1111 is configured for detachable connection to a first mating optical connector 2101 of the medical treatment device 2000. The second optical connector 1121 is configured for detachable connection to a second mating optical connector 2102 of the medical treatment device.

An embodiment of the disposable fiber-optic device 1000 will be described in greater detail below with reference to FIG. 3.

Generally, the medical treatment device 2000 may include a treatment laser source 2200 configured to output treatment laser radiation for treatment of a medical condition. The treatment laser source 2200 may include a suitable type of laser, e.g. a diode laser, configured to emit treatment laser radiation at a wavelength and power suitable for the medical treatment to be performed. When the medical treatment apparatus is for treatment of tumors of the urinary bladder by photocoagulation and/or ablation, the treatment laser source may comprise a high-brightness diode laser. The medical treatment apparatus is configured to output the treatment laser radiation via a cladding, in particular the inner cladding, of the at least doubled-clad optical fiber 1200. To this end, the treatment laser source is optically coupled to the second mating optical connector 2102 for connection to the second optical connector 1121 of the disposable fiber-optic device 1000. Accordingly, the second optical connector is preferably configured to receive a treatment laser beam, in particular a treatment laser beam having an output power of between 10 W and 200 W.

Generally, unless specified otherwise, references to numerical values of the power of laser radiation discussed herein, are intended to refer to the peak power of the laser radiation. For example, when the laser radiation is pulsed, the numerical values correspond to the peak power of the individual pulses rather than on the average power of a pulse train. The latter may be smaller than the peak power and related to the peak power by a factor indicative of the duty cycle of the pulse train.

The medical treatment device 2000 may further comprise a sensor module 2300. The sensor module 2300 may include a sensor laser source 2310 configured to output sensor laser radiation. The medical treatment apparatus is configured to output the sensor laser radiation via a core of the at least doubled-clad optical fiber 1200. To this end, the sensor laser module is optically coupled to the first mating optical connector 2101 for connection to the first optical connector 1111 of the disposable fiber-optic device. The at least one core of the at least double-clad optical fiber may have a V number of less than 10, preferably less than 8, such as less than 6, such as less than 3 or even 2.4, at a wavelength of the sensor laser radiation, i.e. the at least one core may be a few-mode core or even a single-mode core at a wavelength of the sensor laser radiation.

In some embodiments, the sensor laser radiation includes multiple wavelengths. Accordingly, the V number of the least one core of the at least double-clad optical fiber may be different for the different wavelengths of the sensor radiation. For example the core may be a single-mode core at one or some wavelengths of the sensor laser radiation and a few-mode core (or even a multi-mode core) at other wavelengths of the laser radiation. In particular, in some embodiments, the medical treatment apparatus includes more than one sensor module, e.g. an interferometric distance sensor module and a spectroscopic sensor module. The different sensor modules may operate at different wavelengths. In such embodiments, the at least one core of the at least double-clad optical fiber may be a few-mode core with a small V number or even a single-mode core (e.g. having a V number of less than 4, such as less than 3 or even 2.4) at a wavelength of the sensor laser radiation of one of the sensor modules, e.g. of the interferometric sensor module. The at least one core of the at least double-clad optical fiber may be a few-mode core having a larger V number (e.g. between 3 and 10, such as between 3 and 6) at a wavelength of the sensor laser radiation of the other sensor module, e.g. of the spectroscopic sensor module.

The first optical connector is preferably configured to receive a sensor laser beam and to output reflected laser radiation. The sensor module 2300 may further comprise a detector 2320 for detection of radiation received from the tissue to be treated, in particular radiation reflected by the tissue in response to being illuminated by the sensor laser radiation from the sensor laser source 2310. The detector may be configured to receive the radiation from the tissue via the at least double-clad optical fiber 1200 of the disposable fiber-optic device, in particular via the core and/or via a cladding of the at least double-clad optical fiber 1200.

In various embodiments, the medical treatment device comprises an interferometric distance sensor module configured to perform interferometric distance sensing of the distance between the distal end of the at least double-clad optical fiber 1200 and the tissue to be treated. In particular, in some embodiments, the interferometric distance sensor is a common path sensor where the distal end of the optical fiber is operable as a reflector of a reference optical path, thus providing a particularly compact system, which does not require any additional fibers. The medical treatment device is preferably further configured to control the treatment laser radiation responsive to the measured distance.

In some embodiments, the medical treatment device further comprises a spectroscopic sensor for sensing one or more spectral properties of radiation reflected by the tissue to be treated. The medical treatment device is preferably further configured to determine a measure indicative of a status or progress of the treatment from the sensed one or more spectral properties. Preferably, the medical treatment device is configured to control the treatment laser radiation responsive to the determined status or progress, in particular responsive to the sensed distance and the determined status or progress.

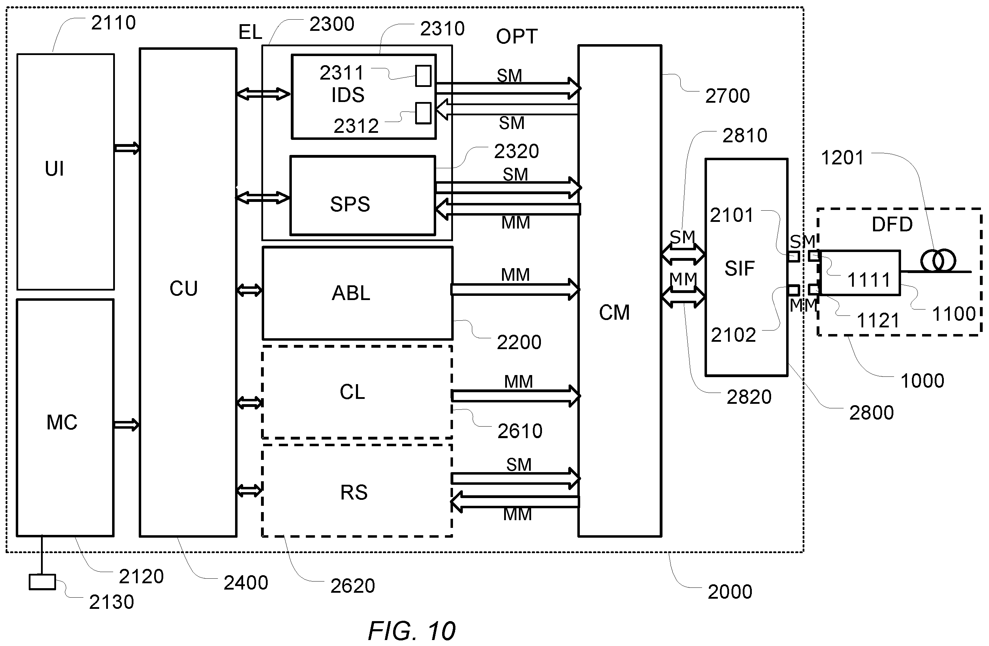

An embodiment of the medical treatment device 2000 will be described in greater detail below with reference to FIG. 10. As will be apparent from the disclosure below, the medical treatment device may include additional or alternative radiation sources and/or additional optical detectors.

In use, the disposable fiber-optic device 1000 is detachably and optically connected to the medical treatment device 2000 via the connector module 1100 and the distal end 1201 of the at least double-clad optical fiber 1200 is inserted into the working channel of an endoscope, e.g. as illustrated in FIG. 2.

FIG. 2 diagrammatically illustrates an example of a disposable fiber-optic device with its at least double-clad optical fiber 1200 having been inserted and advanced into the working channel 3100 of an endoscope 3000. The disposable fiber-optic device comprises an at least double-clad optical fiber 1200 and a connector module 1100, all as described in connection with FIG. 1. In the example of FIG. 2 the distal end 1201 of the at least double-clad optical fiber 1200 extends out of the working channel of the endoscope. In use, the endoscope is inserted into a vessel or organ of a patient, depending on the nature of the medical procedure. The endoscope 3000 may be inserted with the at least double-clad optical fiber 1200 already inserted in the working channel 3100; alternatively, the at least double-clad optical fiber 1200 may be inserted into the working channel 3100 after insertion of the endoscope 3000 into the target organ or vessel.

Endoscopes adapted for various medical procedures are known as such in the art and will not be described in greater detail herein. In particular, different types of endoscopes may have working channels of more or less standardized diameter. Typical working channels of endoscopes have a diameter of between 1 mm and 5 mm. Some endoscopes may further include a camera 3600 and/or one or more illumination lights, e.g. LEDs 3500, arranged at a distal end of the endoscope 3000 so as to allow a physician to observe progress of the medical procedure.

Generally, various embodiments of the disposable fiber-optic device is for treatment of the urinary tract, e.g. for treatment of the urinary bladder, in particular for treatment of bladder cancer.

Accordingly, in some embodiments, the endoscope is a cystoscope adapted for insertion into the urinary bladder through the urinary tract. Working channels of cystoscopes typically have a diameter of between 1.5 mm and 2 mm, such as 1.8 mm. A cystoscope typically has to be able to be bent with a small bending radius, e.g. with a bending radius of 10 mm or less, or even 5 mm or less.

FIG. 3 diagrammatically illustrates an example of a disposable fiber-optic device 1000 in accordance with embodiments disclosed herein.

The disposable fiber-optic device 1000 comprises an at least double-clad optical fiber 1200 having a proximal end and a distal end 1201. The distal end 1201 is configured to be advanced through a working channel of an endoscope, e.g. as described in connection with FIG. 2. The at least double-clad optical fiber 1200 has at least one core, an inner cladding and at least one outer cladding. In particular, the at least double-clad optical fiber 1200 may be a double-clad optical fiber having one or more cores, an inner cladding and an outer cladding.

The disposable fiber-optic device 1000 further comprises a connector module 1100 for optically connecting the proximal end of the at least double-clad optical fiber 1200 to a medical treatment device (not shown in FIG. 3), e.g. as described in connection with FIG. 1.

The connector module 1100 comprises a first optical fiber 1110, a second optical fiber 1120, a first optical connector 1111 for detachably and optically connecting the first optical fiber 1110 to medical treatment device, a second optical connector 1121 for detachably and optically connecting the second optical fiber 1120 to the medical treatment device, and an optical combiner module 1300 configured to couple radiation between the first optical fiber 1110 and the at least one core of the at least double-clad optical fiber 1200 and to couple radiation between the second optical fiber 1120 and at least one cladding of the at least two claddings of the at least double-clad optical fiber 1200.

Generally, detachable connection refers to a connection during normal use performed by a user of the medical treatment apparatus where the connection can be disconnected again during normal use. In particular, detachable connection of an optical connector of the disposable fiber-optic device implies that the corresponding mating connector of the medical treatment device, to which the optical connector is connected, is not destroyed during the connection or disconnection. Preferably, the optical connector of the disposable fiber-optic device is not destroyed during the disconnection either, i.e. the optical connectors of the disposable fiber-optic device can reversibly and repeatedly be connected to the medical treatment device. Optical connection implies that radiation can pass between the medical treatment device and the corresponding optical fiber of the connector module to which the optical connector is coupled.

Generally, in various embodiments, the connector module may comprise a housing 1170. The first optical fiber 1110, the second optical fiber 1120 and the optical combiner module 1300 may completely or partly be accommodated within the housing. The housing 1170 may be a plastic housing or a housing made from another suitable material, e.g. a rigid material or a soft material. The housing may be a box, e.g. made from a rigid material, or it may be provided in another form, e.g. as an elongated flexible sleeve. The housing may form a single compartment or multiple compartments. The housing may have one end configured to be connected to the medical treatment device to thereby connect the first and second optical connectors to corresponding first and second mating optical connectors of the medical treatment device. To this end, the first and/or second optical connector(s), which may be spring-loaded, may be mounted directly on or in a side wall of the housing, thus allowing easy and reliable connection. Alternatively, the first and/or second optical connector(s), which may be spring-loaded, may be mounted to the housing via one or more flexible fiber-optic cables. Accordingly, in general, the components of the connector module may all be accommodated in a single housing or they may distributed between two or more separate housings, which may be interconnected via one or more fiber-optic cables or otherwise.

Generally, the first and/or second optical connector may be spring-loaded. To this end, the first and/or second optical connector may include a spring or other elastic element for holding the first or second optical connector, respectively, in position when connected to the medical treatment device. The spring or other elastic element may be any suitable component for imparting an elastic force, e.g. a flat spring, a coil spring, etc.

The housing may comprise additional mechanical connectors or guides 1171, e.g. guiderails, that further facilitate a reliable and safe connection, e.g. by ensuring fixation to the medical treatment device with a suitable mechanical force on the spring-loaded connectors. To this end, the guiderails or other mechanical connectors may include a click-on mechanism.

The housing 1170 further includes an opening through which the at least double-clad optical fiber 1200 protrudes out of the housing. The housing 1170 may include a soft cone 1172 or other form of stress and/or bending relief at the opening through which the at least double-clad optical fiber 1200 protrudes out of the housing.

The first optical fiber 1110 may be a single-mode optical fiber or an optical fiber (e.g. a double-clad optical fiber) having a single-mode core or it may include a single-mode optical fiber section. The first optical fiber, or at least a core of the first optical fiber, may be single-mode at least at a predetermined target wavelength, in particular the wavelength used by a sensor module of the medical treatment device, e.g. by an interferometric distance sensor module. The first optical fiber 1110 may be a single fiber section or include multiple fiber sections optically coupled to each other, e.g. spliced or otherwise connected to each other.

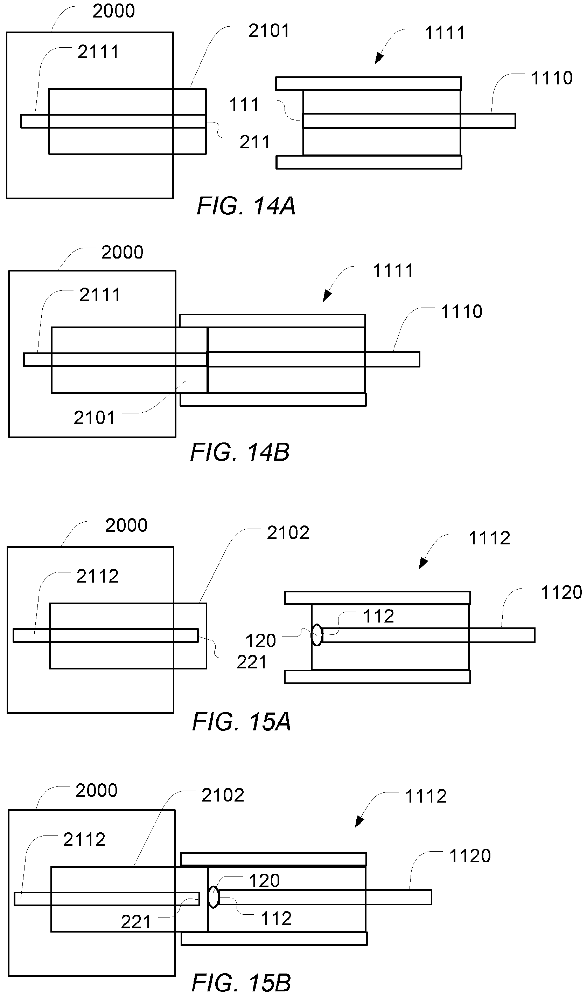

The first optical connector 1111 may be a low-reflectivity optical single-mode fiber connector, such as a connector providing physical contact between respective fiber ends of the optical fibers that are optically connected by the optical connector, i.e. the first optical fiber 1110 and a corresponding first optical fiber of the medical treatment device, e.g. as described in connection with FIGs. 14A and 14B. In particular, the first optical connector may be an Angled Physical Contact (APC) Connector. In some embodiments the low-reflectivity optical single-mode fiber connector has a reflectivity of less than -40 dB, such as - 50 dB, preferably less than -60 dB, such as less than -70 dB, such as -80 dB. Generally, the first optical connector may be configured to couple few-mode or even single-mode laser radiation, in particular radiation characterizable by a V number smaller than 10, such as smaller than 3, into the first optical fiber. The first optical connector may be configured to couple single-mode laser radiation, having a power of less than 1 W, such as less than 200 mW, such as less than 100 mW, into the first optical fiber.

Accordingly, an efficient optical coupling between single-mode or few mode fibers with little back-reflection is provided. This is particularly advantageous when the radiation through the core of the at least double-clad optical fiber 1200 is sensor laser radiation, e.g. for sensor applications, in particular for interferometric measurements such as interferometric distance measurements.

Generally, the terms single-mode radiation or single-mode laser radiation as used herein refer to laser radiation that is transmitted through emitted from a single-mode optical fiber or other single-mode waveguide. Similarly, the terms multi-mode radiation or multi-mode laser radiation as used herein refer to laser radiation that is transmitted through and/or emitted from a multi-mode optical fiber or other multi-mode waveguide.

The second optical fiber 1120 may be a multi-mode optical fiber or include a multi-mode fiber section. The second optical fiber 1120 may be a single fiber section or include multiple fiber sections optically coupled to each other, e.g. spliced or otherwise connected to each other.

The second optical connector 1121 may be a high-power optical multi-mode connector such as an SMA905 connector or a collimated beam connector. A collimated beam connector is a fiber-optic connector including a collimating lens operable to collimate a beam exiting the fiber-optic connector. In particular, the collimating lens may be arranged inside the connector housing of the connector. The collimating lens allows mating of the collimated beam connector with a corresponding collimated beam connector with particularly low power loss. Accordingly, a high-power connection suitable for e.g. high-power treatment laser radiation is provided. The second optical connector may be a free-standing fiber tip connector, i.e. a connector configured for optical coupling without physical contact between the fiber ends of the optically coupled fibers, e.g. as described in connection with FIGs. 15A and 15B. Here, the term high-power optical connector is intended to refer to an optical connector adapted for optical connection of optical fibers carrying high-power laser radiation, in particular laser radiation at powers suitable for medical treatment of tissue, e.g. by photocoagulation and/or ablation. The second optical connector 1121 may be adapted for optical connection of optical fibers carrying laser radiation having a power of 10 W or more, such as 100 W or more, such as 200 W or more, such as between 10 W and 500 W. Generally, the second optical connector may be configured to couple multi-mode laser radiation, in particular radiation characterizable by a V number larger than 10, into the second optical fiber.

Generally, two optical connectors may be operable to be connected with each other via a mating sleeve. Accordingly, in some embodiments, each of the mating optical connectors of the medical treatment device that are operable to be coupled to the optical connectors of the disposable fiber-optic device may comprise or be attached to a respective mating sleeve. The mating sleeve of the second mating optical connector of the medical treatment device that is operable to be coupled to the second optical connector of the disposable fiber-optic device may at least in part be made from a suitable material having a sufficiently high melting point and/or low thermal expansion to allow a high-power optical connection. Examples of suitable materials include Tungsten Carbide.

Generally, the first optical connector 1111 and the second optical connector 1121 provide separate optical coupling of the first optical fiber 1110 and the second optical fiber 1120, respectively, with the medical treatment device, i.e. they may be different connectors. In particular, the first optical connector 1111 provides optical coupling of the first optical fiber 1110 with a corresponding first fiber of the medical treatment device, and the second optical connector 1121 provides optical coupling of the second optical fiber 1120 with a corresponding second fiber of the medical treatment device, the corresponding second fiber of the medical treatment device being different from the corresponding first fiber of the medical treatment device.

In some embodiments, the first and second optical connectors are physically separate connectors. In particular, the optical connector 1111 may have a first connector housing, e.g. including a first ferrule, and the second optical connector 1121 may have a second connector housing, e.g. including a second ferrule, different from the first connector housing. In other embodiments, the first optical connector 1111 and the second optical connector 1121 are accommodated in a single connector housing, or they may otherwise be formed as a single combined connector having multiple optical termini. For example, the single connector housing may be a two-ferrule connector housing, such as a Diamond DM4 connector, having multiple optical termini.

The at least double-clad optical fiber 1200 may feed single-mode, low power sensor laser radiation through its core towards the tissue to be treated. The at least double-clad optical fiber 1200 may further feed single-mode reflected radiation through its core from the distal end of the at least double-clad optical fiber via the first optical connector 1111 to the medical treatment device. The reflected radiation may be sensor laser radiation having been reflected by the distal end 1201 of the at least double-clad optical fiber 1200 and/or sensor laser radiation having been reflected by the tissue to be treated and captured by the distal end 1201 of the at least double-clad optical fiber 1200. Accordingly, the core of the at least double-clad optical fiber 1200 is preferably a single-mode core at least at the wavelength or wavelengths of sensor laser radiation. The at least double-clad optical fiber 1200 may further feed high-power multi-mode treatment laser radiation through its inner cladding towards the tissue to be treated.

For the purpose of the present description, the term "tissue to be treated" is intended to not only refer to the tissue prior to being treated but also to the tissue during the treatment, including to the tissue having already been affected by the treatment. Generally, the tissue to be treated is biological tissue, such as soft tissue.

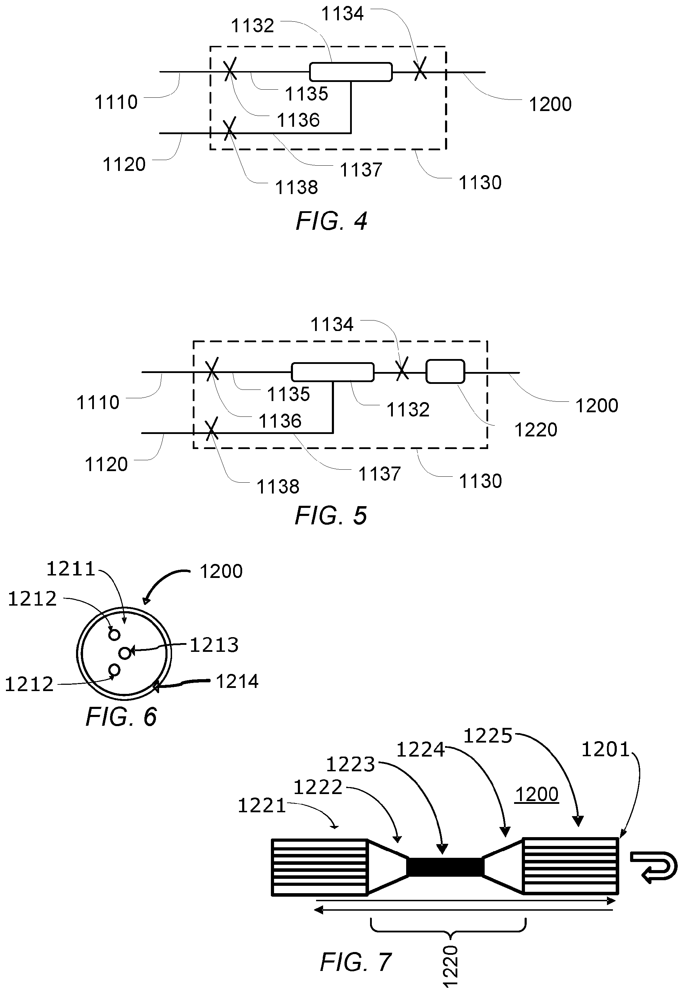

Examples of the optical combiner module 1130 will be described in greater detail with reference to FIGs. 4 and 5, respectively.

Optionally, the connector module 1100 may comprise additional components.

In particular, in some embodiments, the connector module comprises a temperature sensor 1161, e.g. a thermistor, configured to sense the temperature of the optical combiner module 1130. The temperature sensor may be a PTC or NTC sensor. The temperature sensor 1161 may be electrically connectable via electrical connector 1162 to the medical treatment device, thus allowing the medical treatment device to monitor the temperature of the optical combiner module during operation and, optionally, to control operation of the treatment laser source responsive to the sensed temperature. For example, the medical treatment device may be controlled to reduce the power and/or duty-cycle of the treatment laser, or even turn the treatment laser off or close a shutter or optical switch, so as to prevent overheating of the optical combiner module 1130.

Alternatively or additionally, the connector module may comprise a cooling member 1131 configured for passive or active cooling of the optical combiner module 1130. The cooling member may e.g. comprise a heat sink, cooling ribs and/or a thermal conductor configured to transport heat into a heat sink of the medical treatment device when the connector module is connected to the medical treatment device.

Yet alternatively or additionally, the disposable fiber-optic device 1000, in particular the connector module 1100, may include an RFID chip 1140 or other form of device-identifier, e.g. a wirelessly readable ID tag, allowing the medical treatment device to automatically register an identifier of the disposable fiber-optic device 1000. This may be advantageous when the medical treatment device is configured to be operated with different types of disposable fiber-optic devices. It allows the medical treatment device to adapt one or more of its operational parameters to the specific type of disposable fiber-optic device being connected.

FIG. 4 diagrammatically illustrates an example of an optical combiner module 1130 of a connector module of a disposable fiber-optic device in accordance with embodiments disclosed herein.

The optical combiner module 1130 couples radiation between the first optical fiber 1110 of the connector module and a core of the at least double-clad optical fiber 1200 of the disposable fiber-optic device according to an embodiment disclosed herein. The optical combiner module 1130 further couples radiation between the second optical fiber 1120 of the connector module and a cladding, in particular an inner cladding, of the at least double-clad optical fiber 1200.

The first optical fiber 1110 may thus be a single-mode optical fiber, at least at one or more target wavelengths, and the second optical fiber 1120 may be a multi-mode optical fiber.

The optical combiner module 1130 comprises an at least double-clad optical fiber section 1135 having a core and a cladding. The core of the at least double-clad optical fiber section 1135 is a few-mode, preferably a single-mode core at a target wavelength of the radiation received via the first optical fiber. The at least double-clad optical fiber section 1135 may include a low-index acrylate coating, optionally with fluorinated silica (F-SiO2) cladding. The core of the at least double-clad optical fiber section may have a diameter of between 5 $\mu$m and 10 $\mu$m and/or a numerical aperture of between 0.07 and 0.15. The core of the at least double-clad optical fiber section may have a V number at a wavelength $\lambda_{sensor}$ of the sensor laser radiation of V < 10 @ $\lambda_{sensor}$, preferably V < 8 @ $\lambda_{sensor}$, such as V < 7 @ $\lambda_{sensor}$, such as V < 2.4 @ $\lambda_{sensor}$.

The V number is a dimensionless parameter which is often used in the context of optical fibers, such as step-index fibers. It is a normalized frequency parameter, which determines the number of modes of an optical fiber, such as a step-index fiber. It is defined as

$$V = \frac{2\pi}{\lambda} a \, NA = \frac{2\pi}{\lambda} a \sqrt{n_{core}^2 - n_{cladding}^2}$$

where $\lambda$ is the vacuum wavelength, a is the radius of the fiber core and NA is the numerical aperture. $n_{core}$ and $n_{cladding}$ refer to the refractive index of the core and of the inner cladding, respectively.

For the purpose of the present description, and unless otherwise stated, the terms single-mode fiber and single-mode core refer to a fiber and core, respectively, that has a V number less than 2.4 at the relevant wavelength. The terms few-mode fiber and few-mode core refer to a fiber and core, respectively, that has a V number larger than 2.4 and less than 10 at the relevant wavelength. The terms multi-mode fiber and multi-mode core refer to a fiber and core, respectively, that has a V number larger than 10.

The optical combiner module 1130 further comprises a first splice point 1136, which couples radiation between the first optical fiber 1110 and the core of the at least double-clad optical fiber section 1135. The first splice point 1136 may be provided with a cladding mode stripper configured for removal of return multi-mode radiation into the first optical fiber 1110. Alternatively or additionally, the first splice point 1136 may be provided with a scattering surface or a high index glue and/or coating.

The optical combiner module 1130 further comprises a multi-mode fiber section 1137 and a second splice point 1138, which couples radiation between the second optical fiber 1120 and the multi-mode fiber section 1137.

The optical combiner module 1130 further comprises an optical fiber combiner 1132 configured for coupling radiation between the multi-mode fiber section 1137 and the inner cladding of the at least double-clad optical fiber section 1135. The optical fiber combiner 1132 may be a side combiner, sometimes also referred to as a side pump combiner, i.e. a combiner configured to laterally couple radiation between the multi-mode fiber section 1137 and the inner cladding of the at least double-clad optical fiber section 1135 while the core of the at least double-clad optical fiber section passes through the side combiner. Hence, the side combiner couples/fuses a multi-mode secondary fiber (here the multi-mode fiber section 1137) at an angle to the circumferentially outer side of a pass-through primary fiber (here the at least double-clad optical fiber section 1135). Preferably, the secondary fiber has a diameter smaller than the primary fiber. For example, the double-clad feed through fiber may have a diameter of 200 µm (or another suitable diameter) and the secondary fiber may have a fiber diameter of 125 µm (or another suitable diameter smaller than the diameter of the feed-through double-clad fiber). The side coupling may result in an up-conversion of the numerical aperture (NA) of the multi-mode inner cladding of the at least double-clad optical fiber section 1135 and, hence, in the at least double-clad optical fiber 1200, e.g. from NA of 0.22 to about 0.45. This in turn results in a larger spot size of the treatment laser radiation on the tissue to be treated. Moreover, the side combiner allows the sensor laser radiation propagating in the core of the at least double-clad optical fiber section to pass through the side combiner without reflections due to splices or the like.

The optical combiner module 1130 further comprises a third splice point 1134 for coupling the at least double-clad optical fiber section 1135 and the proximal end of the at least double-clad optical fiber 1200, in particular for coupling the cores and claddings of the respective fibers.

FIG. 5 diagrammatically illustrates another example of an optical combiner module 1130 of a connector module of a disposable fiber-optic device in accordance with embodiments disclosed herein. The optical combiner module 1130 is similar to the optical combiner module of FIG. 4 in that it comprises an at least double-clad optical fiber section 1135, a multi-mode fiber section 1137, an optical fiber combiner 1132, a first splice point 1136, a second splice point 1138 and a third splice point 1134, all as described in connection with FIG. 4.

In some embodiments, one or more of the splice points shown in FIGs. 4 and 5 may be omitted. For example, the at least double-clad optical fiber section 1135 may be formed by the proximal end of the at least double-clad optical fiber 1200. Alternatively or additionally, the multi-mode fiber section 1137 may be a portion of the second optical fiber 1120 attached to the second optical connector.

The optical combiner module of FIG. 5 differs from the optical combiner module of FIG. 4 in that it is configured to couple the first and second optical fibers 1110 and 1120, respectively, with a multi-core at least double-clad optical fiber 1200, i.e. the optical combiner module of FIG. 5 is for use with embodiments of a disposable fiber-optic device comprising a multi-core at least double-clad optical fiber.

FIG. 6 diagrammatically illustrates an example of a multi-core double-clad optical fiber 1200. The optical fiber 1200 includes an outer cladding 1214 and an inner cladding 1211, e.g. a multi-mode inner cladding which may be made from quartz. The optical fiber further comprises a central single-mode core 1213 and two single-mode side cores 1212. In other embodiments, the optical fiber may only include a single side core or more than two side cores. Generally, a multi-core optical fiber provides an increased reliability, as it provides multiple redundant paths for the radiation, e.g. sensor laser radiation. This may improve the signal-to-noise ratio of the sensor signals or otherwise improve the reliability of the sensor detection, e.g. allowing continued distance measurements despite parts of the fiber tip being contaminated with tissue or other contaminants.

Again referring to FIG. 5, the optical combiner module 1130 further comprises a tapered fiber region 1220 at the proximal end of the at least double-clad optical fiber 1200. The tapered region 1200 is configured for coupling of radiation between a single core of the at least double-clad optical fiber 1200 and one or more further cores of the at least double-clad optical fiber 1200. The tapered region 1220 may be packaged in a ferrule with air surrounding the taper waist to enable a high numerical aperture so as to support up-converted cladding radiation.

FIG. 7 illustrates operation of the tapered region 1220. In particular, FIG. 7 illustrates the proximal portion 1221 of the at least double-clad optical fiber 1200 extending between the optical fiber combiner 1132 and the tapered fiber region 1220 of the embodiment of FIG. 5, and the distal portion 1225 of the at least double-clad optical fiber 1200 extending out of the housing of the combiner module and being configured for insertion in the working channel of the endoscope. It will be appreciated that FIG. 7 is not drawn to scale and that the distal portion 1225 typically will be much longer than the proximal portion 1221, as the tapered region is preferably

In the example of FIG. 7, the at least double-clad optical fiber 1200 is a multi-core at least double-clad optical fiber with one central core and two side cores, as illustrated in FIG. 6. However, in other embodiments, the multi-core fiber may have a different number of side cores.

During operation, the central core of the proximal fiber section 1221 carries radiation from the first optical fiber 1110. In particular, the radiation may be a single-mode sensor laser radiation, e.g. for interferometric distance measurement and/or other types of sensor radiation, e.g. for spectroscopy. In the proximal fiber section 1221, the side cores preferably carry no or only minimal radiation.

The optical fiber is tapered down in down-taper region 1222 to a reduced-diameter fiber section 1223 and subsequently again tapered up in up-taper region 1224 to its normal diameter in the distal portion 1225.

The down-tapering and subsequent up-tapering enables coupling of radiation from the central core to the side cores. The length of the reduced diameter section 1223 may be chosen so as to enable equal coupling to all cores or so as to couple all radiation from the central core to the side cores. Accordingly, in the proximal portion 1225, the optical fiber 1200 carries radiation from the first optical fiber 1110 either in the side cores only or in all cores.

At the distal end 1201 of the optical fiber 1200, radiation may partially be reflected backwards by the tip. The remaining radiation exits out of the fiber and is reflected back from the tissue towards which the at least double clad optical fiber 1200 is directed. The reflected light may thus again be captured by the at least double-clad optical fiber 1200 and used for sensor purposes, e.g. for interferometric distance sensing of the distance between the distal end 1201 of the at least double-clad optical fiber and the reflecting tissue and/or for spectroscopic analysis of one or more properties of the reflecting tissue.

Reflected light propagating in the reverse direction through the side cores of the distal portion 1225 will then again be concentrated by the tapered region 1220 into the central core of the proximal portion 1221, thus allowing the reflected radiation to be fed back to a medical treatment device via the first optical fiber and the first optical connector of the connector module.

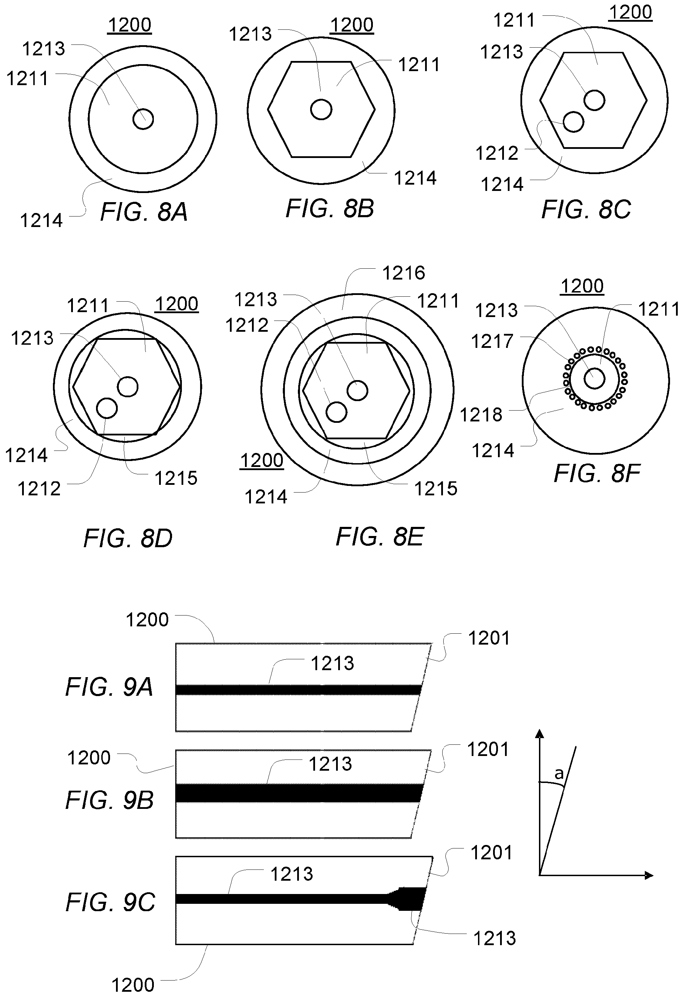

FIGs. 8A-F diagrammatically illustrate further examples of an at least double-clad optical fiber for use in a disposable fiber-optic device in accordance with embodiments disclosed herein.

Generally, in various embodiments, the at least double-clad optical fiber has a core, e.g. a silica core, that is single-mode at least at the wavelength of the sensor laser radiation. The at least double-clad optical fiber has an inner cladding, e.g. a silica cladding, surrounding the core. The inner cladding is operable as a multi-mode core. The at least double-clad fiber further comprises an outer cladding surrounding the inner cladding. The outer cladding may be a silica or polymer cladding, e.g. an FSiO2 layer or a low-index acrylate coating. Some embodiments further comprise an air cladding separating the inner and outer claddings.

The at least double-clad optical fiber may further comprise an outermost protective buffer, e.g. a biocompatible coating which may be made from Nylon, Teflon or other suitable material.

In various embodiments, the refractive index $n_c$ of the core is larger than the refractive index of the inner cladding $n_i$, which in turn is larger than the refractive index $n_o$ of the outer cladding.

In particular, FIG. 8A diagrammatically illustrates a cross-sectional view of an example of a single-core double-clad optical fiber 1200. The single-core double-clad optical fiber 1200 includes an outer cladding 1214 and an inner cladding 1211, e.g. a multi-mode inner cladding which may be made from quartz. The optical fiber further comprises a central single-mode core 1213, e.g. from doped-quartz. In some embodiments, the core may be a few-mode core (e.g. characterizable by a V number smaller than 10).

FIG. 8B diagrammatically illustrates a cross-sectional view of another example of a single-core double-clad optical fiber 1200. As in the previous example, the single-core double-clad optical fiber 1200 includes an outer cladding 1214 and an inner cladding 1211, e.g. a multi-mode inner cladding which may be made from quartz. The optical fiber further comprises a central single-mode core 1213. However, in the present example, the inner cladding has a hexagonal cross section. It will be appreciated that other embodiments may have claddings with other cross sectional shapes, e.g. octagonal. A hexagonal or octagonal cladding or another suitably shaped claddings scrambles modes and enables a more uniform intensity distribution in the multi-mode cladding.

FIG. 8C diagrammatically illustrates a cross-sectional view of yet another example of a double-clad optical fiber 1200. The example is similar to the example of FIG. 8B, except that the double-clad optical fiber of FIG. 8C is a two-core double-clad optical fiber having a side core 1212 in addition to the central core 1213. Both cores may be single-mode or few mode cores.

FIG. 8D diagrammatically illustrates a cross-sectional view of yet another example of an at least double-clad optical fiber 1200. The example is similar to the example of FIG. 8C, except that the optical fiber of FIG. 8D further comprises a down-doped quartz layer 1215 surrounding the shaped cladding 1211. It will be appreciated that the single-core example with a shaped cladding of FIG. 8B may also be provided with such a down-doped quartz layer.

FIG. 8E diagrammatically illustrates a cross-sectional view of yet another example of an at least double-clad optical fiber. The example is similar to the example of FIG. 8D, except that the optical fiber of FIG. 8E further comprises an outer coating/buffer 1216, which may e.g. be made of plastic, EFTE, Teflon, Nylon or another suitable material. It will be appreciated that any of the examples of FIGs. 6, 8A - 8D and 8F may also be provided with such an outer buffer/coating.

In some embodiments, the at least double-clad optical fiber is an air-clad optical fiber. In this respect, FIG. 8F diagrammatically illustrates a cross-sectional view of yet another example of an at least double-clad optical fiber, in particular an example of an air-clad optical fiber. The air-clad fiber comprises a core 1213, an inner cladding 1211, an air cladding 1217, and an outer cladding 1214, where the inner and outer claddings are radially separated by the air cladding. The air cladding 1217 is formed by a plurality of air channels that extend along the longitudinal direction of the fiber. The air channels are distributed around the circumference of the inner cladding 1211.

In various embodiments of an air-clad fiber, the refractive index $n_c$ of the core is larger than the refractive index of the inner cladding $n_i$, which in turn is larger than the refractive index $n_{ac}$ of the air cladding. The refractive index $n_o$ of the outer cladding may be substantially equal to the refractive index $n_i$ of the inner cladding.

Air-clad fibers may be provided with a high numerical aperture, even without the need for special plastic coatings, thus allowing use of soft buffer coatings, which reduces micro-bending losses on the fiber. Large numerical apertures, e.g. numerical apertures of up to 0.65@980 nm, may be provided in some embodiments, thus providing a large spot size of the treatment spot. The air-cladding is also operable as a mode scrambler, thereby providing a more uniform beam profile of the beam exiting the fiber at its distal end.

In some embodiments, the air channels of the air cladding may be collapsed in a short portion, e.g. about 50 $\mu$m or less, at the proximal end of the fiber, thereby reducing multi-mode splicing losses.

In some embodiments, the air channels of the air cladding may be collapsed in a portion, e.g. about 50 $\mu$m or more, at the distal end of the fiber, thereby allowing beam expansion of the multi-mode beam before exiting the fiber.

In some embodiments, the air-clad fiber comprises a fluorine-doped layer 1218 radially inward of the air-cladding, thereby facilitating capturing of more light at the splice points, reducing splice losses and reducing heating issues at the splice points. In some embodiments, the air-clad fiber with fluorine-cladding may have a numerical aperture of between 0.22 and 0.30, matching or exceeding the numerical aperture of the side combiner of the connector module.

Generally, in various embodiments, e.g. in the embodiments of FIGs. 6 and FIGs. 8A-F, the core or cores of the optical fiber may have different dimensions, e.g. as illustrated in FIGs. 9A-C. The choice of core dimensions may strike a balance between bendability of the fiber, signal-to-noise ratio, etc. A large core diameter of e.g. more than 5 $\mu$m, such as between 10 $\mu$m and 15 $\mu$m provides a high signal strength for e.g. an interferometric distance sensor signal. A smaller core diameter of less than 10 $\mu$m, e.g. of about 5 $\mu$m, facilitates tighter bending. In some embodiments, the fiber may have a core diameter of between 5 $\mu$m and 10 $\mu$m with a depressed cladding for improved bending.

FIGs. 9A-C diagrammatically illustrate longitudinal sections of examples of fiber tip designs of an at least double-clad optical fiber for use in a disposable fiber-optic device in accordance with embodiments disclosed herein. Each of FIGs. 9A-C shows a distal portion of the at least double-clad optical fiber 1200 including the distal end 1201. In each of the examples, the distal end is provided as an angled tip, preferably with rounded edges. The tip is angled at an angle a which may e.g. be chosen between 3° and 10°.

Generally, in various embodiments, the optical fiber has a diameter small enough to be advanced through the working channel of an endoscope, such as a cystoscope. In some embodiments, the optical fiber 1200 has a diameter of 2 mm or less, such as 1.5 mm or less, such as 1 mm or less, such as 0.75 mm or less, such as 0.6 mm or less.

For small fibers, it is often not feasible to attach any lenses or other optical elements to the distal end of the fiber, i.e. the optical system is preferably designed to accommodate a situation where the radiation diverges out of the fiber tip towards the tissue to be treated.

FIG. 9A illustrates a single-core fiber having a single-mode core with numerical aperture of 0.14 and a diameter of 6 $\mu$m, thus allowing tight bending.

FIG. 9B illustrates a single-core fiber having a single-mode core with numerical aperture of 0.075 and a diameter of 10 $\mu$m, thus providing a high signal strength for e.g. an interferometric distance sensor signal. Generally, in some embodiments, the at least double-clad optical fiber may have a large core (e.g. with a diameter between 10 $\mu$m and 15 $\mu$m) step index fiber with a surrounding low-index cladding trench, to allow tighter bending.

FIG. 9C illustrates a single-core fiber having a single-mode core with an expanded tip, such as a thermally expanded tip, e.g. expanded from a core diameter of between 5 $\mu$m and 8 $\mu$m, such as 6 $\mu$m, to an expanded core diameter of e.g. 10 $\mu$m. Such a tip design provides a reduced core numerical aperture through a thermal process while causing the fiber mode to expand at the fiber end.

FIG. 10 diagrammatically illustrates a more detailed example of a fiber-optic medical treatment apparatus in accordance with embodiments disclosed herein.

The fiber-optic medical treatment apparatus comprises a medical treatment device 2000 and a disposable fiber-optic device 1000, all as described in connection with FIG. 1. Embodiments of a disposable fiber-optic device are described in connection with FIGs. 2 - 5. The disposable fiber-optic device 1000 is configured to be detachably and optically coupled to the medical treatment device 2000. The disposable fiber-optic device 1000 comprises an at least double-clad optical fiber 1200 and a connector module 1100, all as described in connection with any of the previous figures.

The example fiber-optic medical treatment apparatus is configured for treatment of cancer of the urinary bladder by laser photocoagulation and/or ablation using a cystoscope. It will be appreciated, however, that other embodiments of the fiber-optic medical treatment apparatus may be configured for other types of medical procedures and/or for use with other types of endoscopes.

The medical treatment device 2000 comprises various laser sources, optical detectors and accompanying control circuitry, user interfaces, etc. The medical treatment device 2000 may be embodied with all its modules accommodated in a single housing or with respective modules accommodated in different housings.

The medical treatment device 2000 comprises a user-interface module 2110, e.g. comprising a display, such as a touch-sensitive display and suitable user input devices, such as a keyboard, touch screen etc., allowing an operator to control user-controllable functions of the medical treatment device and to allow the device to output information relevant for the treatment. In some embodiments, the user-interface module comprises an audible and/or visible power/progress indicator configured to output an audible and/or visual indication indicative of a current laser power of the treatment laser radiation or a current status of the treatment. For example the power indicator may indicate the output power of the treatment laser or the measured treatment status, such that the physician knows when to move the fiber to the next spot. An audible indicator may indicate the power level or treatment status by the volume or pitch of the audible signal, by a repetition rate of repeated tones, or in another suitable manner. The medical treatment device 2000 may further comprise one or more control devices 2120, such as a foot pedal, e.g. to allow a physician to activate a treatment laser while manipulating the cystoscope.

The medical treatment device further comprises a control unit 2400 electrically and/or otherwise communicatively connected to the user-interface module 2110 and to the one or more control devices 2120. The control unit 2400 comprises circuitry configured to control various optical treatment and/or sensor modules of the medical treatment device. In particular, the control unit may be configured to control a treatment laser source, a sensor laser source and/or other radiation sources and/or sensors responsive to input received from the user-interface module and/or the one or more control devices. The control unit may further control the treatment laser source, sensor laser source and/or other radiation sources and/or sensors responsive to received sensor signals. The control unit may include suitable drivers and/or communications interfaces and a processing unit, e.g. a suitable programmed central processing unit.

The medical treatment device 2000 comprises an treatment laser source 2200 configured to output treatment laser radiation suitable for treating tumor tissue of the urinary bladder by photocoagulation and/or ablation. In some embodiments, the treatment laser source is configured to output treatment laser radiation at a wavelength which is preferably chosen to provide high water and Hemoglobin absorption and/or low water and high Hemoglobin absorption, such as at about 980 nm or at another suitable wavelength, e.g. in the range between 750 nm and 1000 nm, such as between 790 nm and 820 nm or between 900 nm and 1000 nm, such as between 900 nm and 940 nm, e.g. between 905 nm and 925 nm, or between 970 nm and 1000 nm, such as between 970 nm and 990 nm. The treatment laser radiation may have a suitable power such as between 10 W and 200 W. Treatment laser radiation from the treatment laser source 2200 is preferably fed through the second optical connector 1121 and the cladding of the at least double-clad optical fiber 1200 of the disposable fiber-optic device 1000.

The medical treatment device further comprises a first optical sensor module 2300 configured for interferometric distance sensing of the distance between the distal end of the optical fiber 1200 and the tissue to be treated.

Preferably, the medical treatment device 2000 may be configured to control the treatment laser source 2200 responsive to the sensed distance between the distal end of the optical fiber 1200 and the tissue to be treated, e.g. by reducing the output power of the treatment laser radiation, changing a pulse duration or pulse duty cycle, or even by shutting down the treatment laser radiation when the fiber tip gets close to the tissue. This may prevent unintended damage to the tissue to be treated.

Interferometric distance sensing may be performed using broadband interferometry known as such from optical coherence tomography (OCT). To this end, the first optical sensor module may comprise a suitable sensor laser source 2310 configured to output single-mode laser radiation in a suitable wavelength, e.g. selected in the range between 800 nm and 1100 nm. In applications such as cystoscopy, it is preferred that the wavelength of the sensor laser radiation is chosen at low-water absorption wavelengths, such as wavelengths smaller than 900 nm or wavelength at about 1050 nm. The sensor laser radiation may have an output power of between 1 mW to 500 mW, i.e. considerably smaller, e.g. by at least 2 or 3 orders of magnitude, than the output power of the treatment laser radiation.

The first optical sensor module 2300 further comprise a spectrometer 2320 to perform broadband interferometry. To this end, the first optical sensor module receives reflected radiation back via the disposable fiber-optic device, in particular reference radiation reflected by the tip of the at least double-clad optical fiber 1200 and radiation reflected by the tissue to be treated and captured by the tip of the at least double-clad optical fiber 1200. Based on the reflected radiation, the medical treatment device determines the distance between the distal end of the at least double-clad optical fiber 1200 and the tissue to be treated in a manner known as such in the art. Accordingly, the first optical sensor module 2300 may output sensor laser radiation via the single-mode or few mode core of the at least double-clad optical fiber and receive reflected radiation via the single-mode or few mode core of the at least double-clad optical fiber as a basis for the distance measurement.

Various embodiments of the interferometric distance sensor of the first optical sensor module 2300 provide an axial resolution of between 5 µm and 200 µm, such as between 10 µm and 150 µm, e.g. between 50 µm and 100 µm.

The interferometric distance sensor of the first optical sensor module 2300 may be implemented in different ways. In some embodiments the interferometric distance sensor employs spectral domain OCT using a SLED and a spectrometer. The inventors have found that such a system provides sufficient axial resolution with a bandwidth of the SLED of about 10 nm - 30 nm. Accordingly, the spectrometer only needs to cover a relatively small bandwidth as well. Preferably, the spectrometer has a resolution of between 0.01 nm - 0.05 nm, thereby obtaining a sufficient axial range suitable for distance measurements during treatment. The interferometric distance sensor may operate at wavelengths in the range between about 800 nm and about 900nm where CMOS detectors/spectrometers have good sensitivity and In-GaAs or GaAs SLEDs are readily available. This maximum axial range of the distance sensor may be between about 3 mm to 10 mm, such as between about 3 mm to 9 mm.

In an alternative embodiments, the interferometric distance sensor may employ spectral domain OCT using a swept-source laser and a detector. This embodiment has the advantage of an extended maximum axial range to between about 10 mm and 20 mm. Output powers of 100 mW and higher can be obtained using suitable laser sources, e.g. a 1060 nm InGaAs or GaAs swept source laser of 1-2 mW, and by seeding the laser radiation to a Yb-fiber amplifier or amplifier chain so as to boost the output power to between 2 mW and 500 mW. This embodiment may be implemented similar to conventional swept-source OCT systems, with synchronized data acquisition to the laser sweep. The sweep range may be selected to be between 10 nm and 40 nm, such as about 20 nm or less, and the sweep frequency may be selected to be between 1 kHz and 100 kHz, such as 10 kHz or less.

In order to accommodate measurements via a fiber advanced through a flexible endoscope, which is bent and moved around during treatment, various embodiments of the interferometric distance sensor employ a common path interferometric measurement, where the at least double-clad optical fiber 1200 is used both a reference path and as a sample path. Reflection from the distal end of the at least double clad optical fiber is used as reference signal. Hence, a separate reference arm is avoided, and thereby reducing system complexity and costs.

In such embodiments, a suitable amplitude of the reference signal ensures a good interference signal on the spectrometer. To this end, a suitable reference signal amplitude may be obtained by selecting a suitable fiber tip angle of the distal end of the at least double-clad optical fiber, e.g. an angle between 2 and 10 degrees, depending on the fiber type and laser power.

In various embodiments, the radiation from the sensor laser source 2310 of the interferometric distance sensor 2300 as well as the back-reflected radiation are fed through the single-mode core of the at least double-clad optical fiber 1200 and through the first optical connector 1111 of the disposable fiber-optic device 1000. The inventors have realized that the signal path from the spectrometer of the interferometric distance sensor to the distal end of the at least double-clad optical fiber has low back-reflection losses, preferably less than -60dB. Accordingly, in some embodiments, the first optical connector 1111 of the disposable fiber-optic device, through which the sensor signals for interferometric distance measurement are fed, is preferably a low-reflection connector, e.g. a fiber optical connector with physical contact between fibers, optionally an angled connector such as an E2000 connector.

The medical treatment device 2000 may preferably comprise a second optical sensor module 2500 configured for spectroscopic analysis of the tissue to be treated. In particular, spectral properties of radiation reflected by the tissue to be treated may be used as an indicator of the progress of the treatment. To this end, the medical treatment device may be configured to measure tissue state using reflectance spectroscopy, e.g. using white-light reflectance spectroscopy.

As the tissue is photo-treated, it changes state from an absorbent state to a more scattering state. Low wavelength light will be more reflected than long wavelength light, and by measuring the difference in reflected power at one or more wavelengths, the scattering state of the tissue can be estimated. Measuring the difference in power between two or more wavelengths of back-reflected radiation thus yields a measure of the photocoagulation state of the tissue and, hence, of the status or progress of the treatment. Accordingly, in various embodiments, the medical treatment device is configured to monitor the status of coagulation caused by the treatment laser using reflectance spectroscopy.

In some embodiments, the second sensor module comprises an illumination source using a supercontinuum laser, e.g. covering from a wavelength range between 400 nm and 1100 nm, such as between 400 nm and 800 nm. Alternatively the illumination source may us use one or more single-colored LEDs at respective wavelengths, e.g. at one or more of the following wavelengths: about 400 nm, about 500 nm, about 600 nm and about 700 nm and/or other wavelengths in the range between 400 nm and 700 nm.

In any event, the second optical sensor module 2500 is configured to receive radiation reflected by the tissue to be treated. The second optical sensor module 2500 may thus comprise a suitable detector for measuring the reflected radiation. The detector may e.g. comprise a grating CCD/CMOS spectrometer, e.g. with up to 1 nm resolution, covering a suitable wavelength range, e.g. between 400 nm and 1100 nm, such as between 400 nm and 800 nm. Alternatively, the detector may include a photodetector, which may be time-multiplexed with a plurality of LEDs.

To maintain good signal strength of the radiation reflected back from the tissue, it is preferred that the illumination light passes through the first optical connector 1111 and through the single-mode core of the at least double-clad optical fiber 1200 of the disposable fiber-optic device 1000, i.e. that the illumination light shares the same path through the disposable fiber-optic device as the interferometric distance measurement radiation. Spectroscopy is a non-interferometric method, and hence more limited by noise than an interferometric distance measurement. Therefore, in various embodiments, the multi-mode cladding of the at least double-clad optical fiber 1200 of the disposable fiber-optic device 1000 captures the reflected light of the spectroscopic illumination, i.e. the second optical sensor module 2500 receives radiation reflected back from the tissue to be treated responsive to being illuminated by the illumination source of the second optical sensor module 2500 via the cladding of the at least double-clad optical fiber 1200 and via the second optical connector 1121 of the disposable fiber-optic device 1000.

Accordingly, the medical treatment apparatus may display or otherwise output a progress indication or even automatically control the treatment laser source 2200, e.g. to prevent unintended overtreatment which might otherwise result in undesired tissue damage. In some embodiments, the second optical sensor module 2500 may output sensor laser radiation via the single-mode or few mode core of the at least double-clad optical fiber and receive reflected sensor laser radiation via the single-mode or few mode core or via the multi-mode cladding of the at least double-clad optical fiber. The wavelengths used for spectral analysis may preferably be chosen at low-water absorption wavelengths, such as wavelengths smaller than 900 nm or wavelengths at about 1050 nm. In particular, in some embodiments, the wavelengths used for spectral analysis may preferably be chosen such that they correspond with absorption maxima hemoglobin and/or another suitable molecular marker.

In some embodiments, the medical treatment device may include additional or alternative optical components, e.g. one or more of the following further components: Some embodiments of the medical treatment device 2000 comprise at least one additional treatment laser 2610, e.g. configured for cutting tissue rather than for photocoagulation and/or ablation. The cutting laser may output treatment laser radiation at a suitable wavelength, e.g. infrared radiation, such as at 1940 nm, which is absorbed by water. Treatment laser radiation from the cutting laser 2610 is preferably fed through the second optical connector 1121 and the cladding of the at least double-clad optical fiber 1200 of the disposable fiber-optic device 1000. In some embodiments, the additional treatment laser 2610 outputs treatment laser radiation configured to be absorbed by water in the vicinity of the tissue to be treated and to facilitate removal of treated tissue from the treatment site. To this end, the additional treatment laser 2610 may be configured to output treatment laser radiation at a wavelength between 1000 nm and 2000nm, such as between 1200 nm and 2000 nm.

Alternatively or additionally, the medical treatment device 2000 may comprise a Raman sensor 2620 or other suitable sensor configured for determining properties of a tumor to be treated. The Raman sensor may e.g. employ a 785 nm 300 mW Raman laser. Sensor laser radiation from the Raman laser is preferably fed through the first optical connector 1111 and the single-mode core of the at least double-clad optical fiber 1200 of the disposable fiber-optic device 1000. Return radiation to the Raman sensor is preferably fed through the cladding of the at least double-clad optical fiber 1200 and the second optical connector 1121 of the disposable fiber-optic device 1000.

Yet alternatively or additionally, the medical treatment device may include a pilot light source, e.g. a pilot laser source or other type of light source adapted to emit visible light suitable as pilot beam to illuminate the portion of the tissue to be treated and to serve as aiming guide to the physician or other user of the apparatus. The pilot beam may be fed through the at least double-clad optical fiber 1200, e.g. through the core and/or the inner cladding of the at least double-clad optical fiber 1200. In some embodiments, the pilot beam may be multi-colored. In particular, the pilot beam may include two or more color components detectable by a camera of the medical treatment apparatus, e.g. including at least two detectable color components that are at least 40 nm apart. The at least two color components may be part of a broad-band emission spectrum having a bandwidth of 40 nm or more, where the intensity of the emitted pilot light may be substantially uniform across the bandwidth or vary across the bandwidth. The two detectable color components may each have an intensity high enough for them to be detectable by a camera or similar detector of the medical treatment apparatus such that the spot illuminated by the pilot beam is visible to the human observer in a camera image as an illuminated spot having a color resulting as a mix of said at least two color components, e.g. as a white or substantially white spot.

In some embodiments, the apparatus is configured to control a visible attribute, in particular an intensity, a color and/or color distribution, of the visible pilot light responsive to one or more of: a user command, an operational parameter of the first treatment laser radiation and a sensor signal obtained by a sensor module. For example, the apparatus may control the visible attribute of the pilot beam, e.g. change a color and/or intensity of the pilot beam, responsive to the user operating a foot pedal or other input device for activating the treatment laser radiation. Alternatively or additionally, the apparatus may control the visible attribute responsive to one or more operational parameter of at least the first setting, e.g. a selected laser power and/or duty cycle and/or whether the apparatus is currently emitting the first or second treatment laser radiation. Yet alternatively or additionally, the apparatus may control the visible attribute responsive to one or more sensor signals, e.g. responsive to a sensed distance between the distal end and the tissue to be treated, a detected tissue property. The detected tissue property may e.g. be indicative of the presence of tissue within a range of the distal end and/or a sensed stage of photocoagulation or ablation and/or the like. The tissue property may be sensed by sensing one or more spectral properties of radiation reflected by the tissue to be treated or otherwise. Accordingly, the intensity, color or other visible property of the pilot beam may be used to indicate one or more conditions or operational parameters to the user.

In some embodiments, the medical treatment apparatus comprises a handheld communication device 2130. The handheld communication device may be communicatively coupled to the medical treatment device 2000, e.g. via a wired or wireless connection. The handheld communication device comprises a user input device, e.g. a push button, that is configured to receive user input from a patient during treatment with the medical treatment apparatus. The user input may be indicative of pain or discomfort experienced by the patient during the treatment. To this end the patient may be instructed to press a button if the patient experiences pain or discomfort during the treatment. In some embodiments, the user input device may be configured to measure a force or pressure applied to the user input. The medical treatment device may thus derive a degree of pain or discomfort from the measured force or pressure. The control unit 2400 of the medical treatment device may thus be configured to select a laser power or a pulse duration or another operational parameter of the treatment laser radiation at least in part responsive to the received user input.

The medical treatment device 2000 further comprises an optical combiner module 2700 having optical interfaces to the treatment laser source 2200, the first optical sensor module 2300 with an interferometric distance sensor, and the optional second optical sensor 2500. In embodiments with additional optical treatment and/or sensor units, the optical combiner module also has optical interfaces to any such units, e.g. to a cutting laser 2610 and/or a Raman sensor 2620.

As was described above, the treatment laser source 2200 emits relatively high-power treatment laser radiation to be fed through the multi-mode cladding of the at least double-cad optical fiber while the interferometric distance sensor of the first optical sensor module 2300 emits and receives low-power radiation via the single-mode or few-mode core of the at least double-cad optical fiber. The second optical sensor module 2500 emits radiation to be fed through the single-mode or few-mode core of the at least double-clad optical fiber 1200 of the disposable fiber-optic device, and receives radiation through the cladding of the at least double-clad optical fiber 1200 of the disposable fiber-optic device.

Accordingly, the optical combiner module 2700 is configured to combine the optical paths fed through the core of the at least double-clad optical fiber 1200 via the first optical connector 1111 of the disposable fiber-optic device 1000 with one another. The optical combiner module 2700 is further configured to combine the optical paths fed through the cladding of the at least double-clad optical fiber 1200 via the second optical connector 1121 of the disposable fiber-optic device 1000. To this end, the optical combiner module 2700 may comprise suitable fused fiber combiners for coupling the signal paths fed through the single-mode core of the at least double-clad optical fiber 1200. Similarly, the optical combiner module may comprise suitable free-space components, lenses and dichroic filters to perform multi-mode power and wavelength splitting/combination of the radiation fed through the cladding of the at least double-clad optical fiber 1200.

The optical combiner module 2700 comprises two optical interfaces to be coupled to the disposable fiber-optic device 1000: a single-mode or few-mode interface 2810 (e.g. characterizable by a V number smaller than 10) and a multi-mode interface 2820 (e.g. characterizable by a V number larger than 20), respectively.

The medical treatment apparatus preferably comprises a sacrificial interface module 2800 having corresponding optical interfaces to interface with the single-mode or few-mode interface 2810 and the multi-mode interface 2820, respectively. The sacrificial interface module 2800 optically connects the single-mode or few-mode interface 2810 with a first mating optical connector 2101 to which the first optical connector 1111 of the disposable fiber-optic device 1000 is connectable. Moreover, the sacrificial interface module 2800 optically connects the multi-mode interface 2820 with a second mating optical connector 2102 to which the second optical connector 1121 of the disposable fiber-optic device 1000 is connectable. During operation of the medical treatment apparatus, different disposable fiber-optic devices may interchangeably be connected to the mating first and second optical connectors 2101 and 2102, respectively, of the sacrificial interface module 2800, as the disposable fiber-optic device 1000 is typically a single-use device that is replaced by a new disposable fiber-optic device between each treatment. Therefore, the first and second mating optical connectors of the sacrificial interface may be subject to wear. Accordingly, provision of a sacrificial interface module 2800 that can relatively easily be replaced facilitates maintenance of the medical treatment device 2000.

It will be appreciated that the radiation form/to the respective optical modules of the medical treatment device may not necessarily be fed through the disposable fiber-optic device concurrently. Instead, they may be time-multiplexed in a suitable manner to avoid that they interact with each other in an undesired manner.

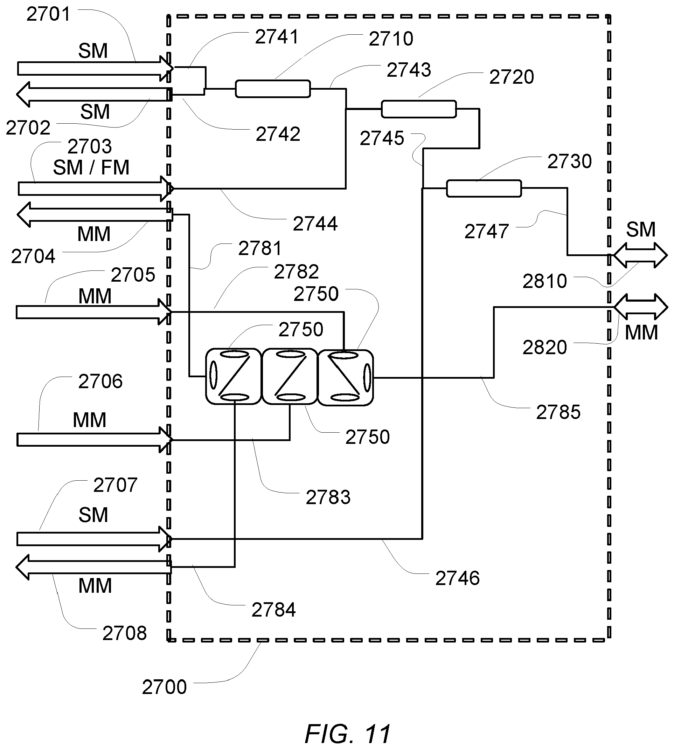

FIG. 11 diagrammatically illustrates an example of an optical combiner module for combining multiple optical paths to be fed through an at least double-clad optical fiber. The optical combiner module 2700 has optical interfaces 2701-2708 to the various optical components of the medical treatment device, in particular to the treatment laser source, and to the sensor module.

In the illustrated embodiment, the optical combiner module 2700 has an optical interface 2705 for receipt of multi-mode treatment laser radiation from the treatment laser source of the medical treatment device. The optical combiner module 2700 further has an optical interface 2701 for receipt of single-mode sensor laser radiation from the interferometric distance sensor module of the medical treatment device and an optical interface 2702 for feeding single-mode response radiation, which has been reflected by the tissue to be treated in response to being illuminated by the sensor laser radiation from the interferometric distance sensor module, back to the interferometric distance sensor module of the medical treatment device. Similarly, in embodiments, where the medical treatment device further comprises a spectroscopic sensor module, the optical combiner module 2700 further has an optical interface 2703 for receipt of single-mode or few-mode sensor laser radiation from the spectroscopic sensor module of the medical treatment device and an optical interface 2704 for feeding multi-mode response radiation, which has been reflected by the tissue to be treated in response to being illuminated by the sensor laser radiation from the spectroscopic sensor module, back to the spectroscopic sensor module of the medical treatment device.

Moreover, in embodiments, where the medical treatment device further comprises a further treatment laser source, e.g. a cutting laser, the optical combiner module 2700 further has an optical interface 2706 for receipt of multi-mode treatment laser radiation from the additional treatment laser source of the medical treatment device. Similarly, in embodiments, where the medical treatment device further comprises an additional sensor module, e.g. a Raman sensor, the optical combiner module 2700 further has an optical interface 2707 for receipt of single-mode or few-mode sensor laser radiation from the additional sensor module of the medical treatment device and an optical interface 2708 for feeding multi-mode response radiation, which has been reflected by the tissue to be treated in response to being illuminated by the sensor laser radiation from the additional sensor module, back to the additional sensor module of the medical treatment device. It will be appreciated that alternative embodiments of the combiner module 2700 may have fewer, additional and/or alternative optical interfaces to fewer, additional and/or alternative optical modules of the medical treatment apparatus.

The optical combiner module 2700 is configured to combine the optical paths fed through the core of the at least double-clad optical fiber with one another. The optical combiner module 2700 is further configured to combine the optical paths fed through the inner cladding of the at least double-clad optical fiber.

To this end, the optical combiner module 2700 comprises suitable fused fiber combiners for coupling the signal paths fed through the single-mode core of the at least double-clad optical fiber 1200. In particular, the optical combiner module 2700 comprises a first fused fiber combiner 2710, e.g. a single-mode power splitter, which is connected to optical interfaces 2710 and 2702 via respective single-mode optical fibers 2741 and 2742, respectively. The optical combiner module 2700 further comprises a second fused fiber combiner 2720, e.g. configured for wavelength multiplexing, which is connected to the first fused fiber combiner 2710 via single-mode fiber 2743 and connected to optical interface 2703 via single-mode optical fiber 2744. The second fused fiber combiner 2720 may be operable to perform wavelength multiplexing between single-mode radiation at the wavelength of the interferometric distance sensor and few-mode radiation at the wavelengths of the spectroscopic sensor module. In embodiments with an additional sensor module, the optical combiner module 2700 may further comprise a third fused fiber combiner 2730, e.g. configured for wavelength multiplexing, which is connected to the second fused fiber combiner 2720 via single-mode fiber 2745 and connected to optical interface 2707 via single-mode optical fiber 2746. The combined single-mode and few-mode radiation is output by the optical combiner module via single mode fiber 2747. The single-mode fibers 2741-2747 are single-mode at least at the wavelength of the sensor laser radiation of the interferometric sensor module. The single-mode fibers 2741-2747 may have cores having a diameter of less than 15 $\mu$m, such as less than 10 $\mu$m. The single-mode fibers 2741-2747 may have a V number of less than 10 at the respective wavelengths being combined.

The optical combiner module 2700 further comprises suitable free-space components 2750, e.g. including lenses and dichroic filters, to perform multi-mode power and wavelength splitting/combination of the radiation fed through the inner cladding of the at least double-clad optical fiber. To this end, the free-space components are optically coupled via respective multi-mode fibers 2781-2784 to optical interfaces 2704, 2705, 2706 and 2708, respectively. The combined multi-mode radiation is output by the optical combiner module via multi-mode fiber 2785. The multi-mode fibers 2781-2785 may have cores of diameters between 30 μm and 500 μm, such as between 50 μm and 400 μm, such as between 100 μm and 200 μm. The multi-mode fibers 2781-2785 may have a V number of 20 or more.

Accordingly, the optical combiner module 2700 comprises two optical interfaces to be coupled to the disposable fiber-optic device: a single-mode or few-mode interface 2810 (e.g. characterizable by a V number smaller than 10) and a multi-mode interface 2820 (e.g. characterizable by a V number larger than 20), respectively.

Generally, in various embodiments, the medical treatment apparatus comprises an optical combiner module comprising one or more fused fiber couplers configured to multiplex sensor laser radiation from the interferometric distance sensor with sensor laser radiation from the spectroscopic sensor in to an optical fiber that is single-mode at least at a wavelength of the sensor laser radiation of the interferometric distance sensor. In particular, the wavelength(s) of the sensor laser radiation of the interferometric distance sensor may be different from the wavelength(s) of the spectroscopic sensor. The fiber couplers may be fused 3 dB couplers.

In various embodiments, the medical treatment apparatus comprises an optical combiner module configured to multiplex multi-mode treatment laser radiation and multi-mode sensor radiation into a multi-mode optical fiber. To this end, the optical combiner module may include free space components including one or more lenses and one or more dichroic mirrors. The multi-mode sensor radiation may include multi-mode response radiation, which has been reflected by the tissue to be treated in response to being illuminated by sensor laser radiation emitted by the spectroscopic sensor module.

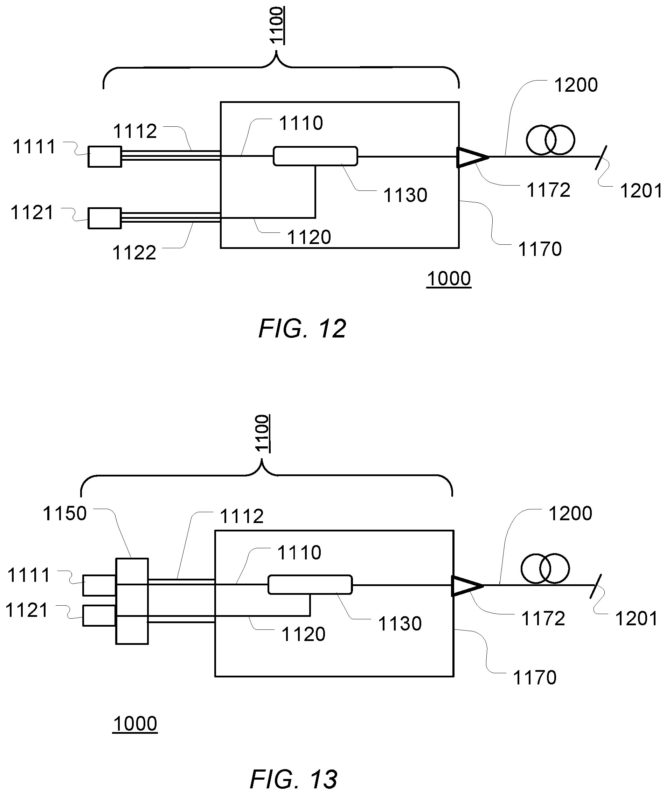

FIG. 12 diagrammatically illustrates another example of a disposable fiber-optic device 1000 in accordance with embodiments disclosed herein.

The disposable fiber-optic device 1000 is similar to the optical fiber device described in connection with FIG. 3 in that it comprises an at least double-clad optical fiber 1200 having a proximal end and a distal end 1201, all as described in connection with FIG. 3.

The disposable fiber-optic device 1000 further comprises a connector module 1100 for optically connecting the proximal end of the at least double-clad optical fiber 1200 to a medical treatment device (not shown in FIG. 3), e.g. as described in connection with FIG. 1.

The connector module 1100 comprises a first optical fiber 1110, a second optical fiber 1120, a first optical connector 1111 for detachably and optically connecting the first optical fiber 1110 to medical treatment device, a second optical connector 1121 for detachably and optically connecting the second optical fiber 1120 to the medical treatment device, and an optical combiner module 1300 configured to couple radiation between the first optical fiber 1110 and the at least one core of the at least double-clad optical fiber 1200 and to couple radiation between the second optical fiber 1120 and at least one cladding of the at least two claddings of the at least double-clad optical fiber 1200, all as described in connection with FIG. 3.

The connector module 1170 further comprises a housing 1170, where a portion of the first optical fiber 1110, at portion of the second optical fiber 1120 and the optical combiner module 1300 are accommodated within the housing. The connector module 1100 of FIG. 12 differs from the example of FIG. 3 in that the first optical connector 1111 and the second optical connector 1121 are not integrated into the housing 1170 but are connected to the housing 1170 via flexible fiber-optic cables 1112 and 1122, respectively, through which the first and second optical fibers 1110 and 1120, respectively extend. In the example of FIG. 12, the first and second optical connectors 1111 and 1121 each have their separate connector housing.

FIG. 13 diagrammatically illustrates another example of a disposable fiber-optic device 1000 in accordance with embodiments disclosed herein.

The disposable fiber-optic device 1000 is similar to the optical fiber device described in connection with FIG. 13, except that the first and second optical connectors 1111 and 1121 are accommodated in a common connector housing 1150, which is connected to the housing 1170, which accommodates the optical combiner 1130, via a flexible fiber-optic cable 1112 through which the first optical fiber 1110 and the second optical fiber 1120 extend. It will be appreciated that, in alternative examples, the connector housing 1150 may be connected to housing 1170 via two separate fiber-optic cables for the first and second optical fibers, respectively.

It will be appreciated that, even though not explicitly shown or described, each of the examples of FIGs. 12 and 13 may include one or more of the additional components described in connection with FIG. 3, e.g. a temperature sensor, an RFID chip and/or a cooling member. Similarly, the connector housing 1150 of the example of FIG. 13 may include mechanical connectors or guides as described in connection with the housing of FIG. 3.

FIGs. 14A and 14B schematically illustrate an example of a first optical connector of an embodiment of a disposable fiber-optic device as disclosed herein, in particular a low-reflectivity optical single-mode fiber connector. The first optical connector 1111 is for detachably and optically connecting the first optical fiber 1110 of the fiber-optic device to a medical treatment device 2000, in particular to a mating optical connector 2101 of the medical treatment device 2000. FIG. 14A illustrates the first optical connector in a non-connected state, while FIG. 14B illustrates the first optical connector 1111 in a connected state, i.e. where the first optical connector 1111 is detachably connected to the mating optical connector 2101 and the first optical fiber 1110 is optically coupled to an optical fiber 2111 of the medical treatment device 2000. The first optical connector 1111 is configured to optically couple the proximal end 111 of the first optical fiber 1110 to an end facet 211 of the optical fiber 2110 of the medical treatment device 2000, when the first optical connector 1111 is detachably connected to the mating optical connector 2101. In particular, the first optical connector 1111 is a physical contact connector, i.e. an optical connector that is configured to detachably connect the proximal end 111 of the first optical fiber 1110 to the medical treatment device 2000 with the proximal end 111 of the first optical fiber 1110 abutting the end facet 211 of the optical fiber 2111 of the medical treatment device. To this end, the end facets of the first optical fiber and of the optical fiber of the medical treatment device may be slanted and/or rounded, so as to provide an improved optical coupling when being brought into physical contact. The first optical fiber and/or the optical fiber of the medical treatment device may be mounted spring-loaded into their respective connector housings so as to ensure physical contact between the fiber ends. It will be appreciated that the optical fibers may be circumferentially surrounded by respective ferrules. The ferrules are preferably configured such that the fiber ends extend at least o the axial ends of the respective ferrules so as to ensure physical contact of the fiber end facets when the first optical connector is detachably connected to the mating optical connector 2101.

FIGs. 15A and 15B schematically illustrate an example of a second optical connector of an embodiment of a disposable fiber-optic device as disclosed herein, in particular a high-power optical multi-mode connector. The second optical connector 1112 is for detachably and optically connecting the second optical fiber 1120 of the fiber-optic device to a medical treatment device 2000, in particular to a mating optical connector 2102 of the medical treatment device 2000. FIG. 15A illustrates the second optical connector in a non-connected state, while FIG. 15B illustrates the second optical connector 1112 in a connected state, i.e. where the second optical connector 1112 is detachably connected to the mating optical connector 2102 and the second optical fiber 1120 is optically coupled to an output optical fiber 2112 of the medical treatment device 2000. The second optical connector 1112 is configured to optically couple the proximal end 112 of the second optical fiber 1120 to an end facet 221 of the output optical fiber 2112 of the medical treatment device 2000, when the second optical connector 1112 is detachably connected to the mating optical connector 2102. In particular, the second optical connector 1112 is a free-standing fiber tip connector, i.e. a connector configured for optical coupling without physical contact between the fiber ends of the optically coupled fibers. The second optical connector 1112 is configured to detachably connect the proximal end 112 of the second optical fiber 1120 to the medical treatment device 2000 with the proximal end 111 of the second optical fiber 1120 positioned at a distance from an end facet 221 of the output optical fiber 2112 of the medical treatment device 2000. To this end the second optical connector 1112 may include a lens 120, such as a collimating lens, configured to couple light emitted from the end facet 221 of the output optical fiber 2112 into the end facet 112 of the second optical fiber 1120. Alternatively or additionally, the mating optical connector 2101 may include one or more lenses. It will be appreciated that the optical fibers may be circumferentially surrounded by respective ferrules. Generally, the second optical connector is configured, when detachably connected to the medical treatment device, to allow light to be coupled into the second optical fiber without bringing the proximal end of the second optical fiber into direct physical contact with an optical component of the medical treatment device. It will be appreciated that the space separating the proximal end 112 of the second optical fiber 1120 and the end facet 112 of the output optical fiber 2112 may be a void filled by air or by another material that is suitably transparent for the laser radiation transmitted via the second optical fiber.

Various aspects have been described with reference to various embodiments. Modifications and alterations will occur to others upon reading the present disclosure. It is intended that the invention be construed as including all such modifications and alterations, including insofar as they come within the scope of the appended claims.

## Claims

1. A disposable fiber-optic device, wherein the disposable fiber-optic device comprises:

    - an at least double-clad optical fiber (1200) having a proximal end and a distal end, the distal end being configured to be advanced through a working channel of an endoscope, wherein the at least double-clad optical fiber has at least one core and at least two claddings; and
    - a connector module (1100) for optically connecting the proximal end of the at least double-clad optical fiber to a medical treatment device, wherein the connector module comprises:

        - a first optical fiber (1110),
        - a second optical fiber (1120),
        - a first optical connector (1111) for detachably and optically connecting the first optical fiber to the medical treatment device,
        - a second optical connector (1121) for detachably and optically connecting the second optical fiber to the medical treatment device, and
        - an optical combiner module (1130) configured to couple radiation between the first optical fiber and the at least one core of the at least double-clad optical fiber and to couple radiation between the second optical fiber and at least one cladding of the at least two claddings of the at least double-clad optical fiber.

2. A disposable fiber-optic device according to claim 1, wherein the first optical connector is a low-reflectivity optical single-mode connector providing physical contact between respective fiber ends of the optical fibers that are optically connected by the first optical connector, and wherein the second optical connector is a free-standing fiber tip connector for high-power optical multi-mode connection.

3. A disposable fiber-optic device according to any one of the preceding claims, wherein the first and/or second optical connectors is spring-loaded.

4. A disposable fiber-optic device according to any one of the preceding claims, wherein the optical combiner module includes a side combiner configured to laterally couple radiation between the second optical fiber, or a multimode fiber section optically coupled to the second optical fiber, and the at least one cladding of the at least double-clad optical fiber, and wherein the at least one core of the at least double-clad optical fiber passes through the side combiner.

5. A disposable fiber-optic device according to any one of the preceding claims, wherein the optical combiner module includes a side combiner configured to laterally couple radiation between a multimode fiber section having a first fiber diameter and at least one cladding of an at least double-clad optical fiber, wherein at least one core of the at least double-clad optical fiber passes through the side combiner and wherein the double-clad optical fiber has a diameter larger than the diameter of the multimode fiber section.

6. A disposable fiber-optic device according to any one of the preceding claims, wherein the connector module comprises a temperature sensor and/or a cooling member configured for cooling the optical combiner module.

7. A disposable fiber-optic device according to any one of the preceding claims, wherein the at least double-clad optical fiber is a multicore fiber having two or more cores, and wherein the at least double-clad optical fiber comprises a tapered region configured for coupling of radiation between a first core of the two or more cores and at least a second core of the two or more cores, different from the first core.

8. A disposable fiber-optic device according to any one of the preceding claims, wherein distal end of the at least double-clad optical fiber has an angled tip.

9. A disposable fiber-optic device according to any one of the preceding claims, wherein the connector module comprises a first housing accommodating at least the optical combiner module and at least a portion of the first optical fiber and at least a portion of the second optical fiber.

10. A disposable fiber-optic device according to claim 9, wherein the first housing further accommodates the first and second optical connectors, and/or wherein the first optical connector and/or the second optical connector is/are arranged outside of the first housing and connected to the first housing via a flexible fiber-optic cable accommodating a portion of the first optical fiber and/or the second optical fiber.

11. A disposable fiber-optic device according to any one of the preceding claims, wherein the first optical fiber has a proximal end and a distal end, wherein the first optical connector is configured to optically couple the proximal end of the first optical fiber to an end face of an optical fiber of the medical treatment device, and wherein the first optical connector is configured to detachably connect the proximal end of the first optical fiber to the medical treatment device with the proximal end of the first optical fiber abutting the end face of the optical fiber of the medical treatment device.

12. A disposable fiber-optic device according to any one of the preceding claims, wherein the first optical connector is configured to couple single-mode laser radiation having a V number smaller than 10, such as smaller than 3.

13. A disposable fiber-optic device according to any one of the preceding claims, wherein the second optical fiber has a proximal end and a distal end, wherein the second optical connector is configured to optically couple the proximal end of the second optical fiber to an output optical fiber of the medical treatment device, and wherein the second optical connector is configured to detachably connect the proximal end of the second optical fiber to the medical treatment device with the proximal end of the second optical fiber positioned at a distance from an end facet of the output optical fiber of the medical treatment device.

14. A disposable fiber-optic device according to any one of the preceding claims, wherein the second optical connector is configured to couple multi-mode laser radiation having a V number larger than 10 and a power of 10 W or more into the second optical fiber.

15. A fiber-optic medical treatment apparatus comprising a medical treatment device and a disposable fiber-optic device according to any one of the preceding claims, wherein the disposable fiber-optic device is configured to be detachably and optically coupled to the medical treatment device.

**Patentansprüche**

1. Einweg-Faseroptikvorrichtung, wobei die Einweg-Faseroptikvorrichtung Folgendes umfasst:

   - eine mindestens doppelmantelige optische Faser (1200), die ein proximales Ende und ein distales Ende aufweist, wobei das distale Ende konfiguriert ist, durch einen Arbeitskanal eines Endoskops vorgeschoben zu werden, wobei die mindestens doppelmantelige optische Faser mindestens einen Kern und mindestens zwei Mäntel aufweist; und
   - ein Verbindungsmodul (1100) zum optischen Verbinden des proximalen Endes der mindestens doppelmanteligen optischen Faser mit einer medizinischen Behandlungsvorrichtung, wobei das Verbindungsmodul Folgendes umfasst:

     - eine erste optische Faser (1110),
     - eine zweite optische Faser (1120),
     - einen ersten optischen Steckverbinder (1111) zum lösbaren und optischen Verbinden der ersten optischen Faser mit der medizinischen Behandlungsvorrichtung,
     - einen zweiten optischen Steckverbinder (1121) zum lösbaren und optischen Verbinden der zweiten optischen Faser mit der medizinischen Behandlungsvorrichtung, und
     - ein optisches Kombinatormodul (1130), das konfiguriert ist, Strahlung zwischen der ersten optischen Faser und dem mindestens einen Kern der mindestens doppelmanteligen optischen Faser einzukoppeln und Strahlung zwischen der zweiten optischen Faser und mindestens einem Mantel der mindestens zwei Mäntel der mindestens doppelmanteligen optischen Faser einzukoppeln.

2. Einweg-Faseroptikvorrichtung nach Anspruch 1, wobei der erste optische Steckverbinder ein optischer Singlemode-Steckverbinder mit geringer Reflektivität ist, der einen physikalischen Kontakt zwischen den jeweiligen Faserenden der optische Fasern bereitstellt, die durch den ersten optischen Steckverbinder optisch verbunden sind, und wobei der zweite optische Steckverbinder ein freistehender optischer Spitzen-Steckverbinder für optische Multimode-Hochleistungsverbindungen ist.

3. Einweg-Faseroptikvorrichtung nach einem der vorstehenden Ansprüche, wobei der erste und/oder zweite optische Steckverbinder federbelastet ist.

4. Einweg-Faseroptikvorrichtung nach einem der vorstehenden Ansprüche, wobei das optische Kombinatormodul einen Seitenkombinator einschließt, der konfiguriert ist, Strahlung seitlich zwischen der zweiten optischen Faser oder einem Multimode-Faserabschnitt, der optisch mit der zweiten optischen Faser gekoppelt ist, und dem mindestens einen Mantel der mindestens doppelmanteligen optische Faser einzukoppeln, und wobei der mindestens eine Kern der mindestens doppelmanteligen optischen Faser durch den Seitenkombinator verläuft.

5. Einweg-Faseroptikvorrichtung nach einem der vorstehenden Ansprüche, wobei das optische Kombinatormodul einen Seitenkombinator einschließt, der konfiguriert ist, Strahlung seitlich zwischen einem Multimode-Faserabschnitt, der einen ersten Faserdurchmesser aufweist, und mindestens einen Mantel einer mindestens doppelmanteligen optischen Faser einzukoppeln, wobei mindestens ein Kern der mindestens doppelmanteligen optischen Faser durch den Seitenkombinator verläuft und wobei die doppelmantelige optische Faser einen Durchmesser aufweist, der größer ist als der Durchmesser des Multimode-Faserabschnitts.

6. Einweg-Faseroptikvorrichtung nach einem der vorstehenden Ansprüche, wobei das Verbindungsmodul einen Temperatursensor und/oder ein Kühlelement umfasst, das zur Kühlung des optischen Kombinatormoduls konfiguriert ist.

7. Einweg-Faseroptikvorrichtung nach einem der vorstehenden Ansprüche, wobei es sich bei der mindestens doppelmanteligen optischen Faser um eine Mehrkernfaser handelt, die zwei oder mehr Kerne aufweist, und wobei die mindestens doppelmantelige optische Faser einen verjüngten Bereich aufweist, der zur Einkopplung von Strahlung zwischen einem ersten Kern der zwei oder mehr Kerne und mindestens einem zweiten Kern der zwei oder mehr Kerne, der sich vom ersten Kern unterscheidet, konfiguriert ist.

8. Einweg-Faseroptikvorrichtung nach einem der vorstehenden Ansprüche, wobei das distale Ende der mindestens doppelmanteligen optische Faser eine abgewinkelte Spitze aufweist.

9. Einweg-Faseroptikvorrichtung nach einem der vorstehenden Ansprüche, wobei das Verbindungsmodul ein erstes Gehäuse umfasst, das mindestens das optische Kombinatormodul und mindestens einen Abschnitt der ersten optischen Faser und mindestens einen Abschnitt der zweiten optischen Faser aufnimmt.

10. Einweg-Faseroptikvorrichtung nach Anspruch 9, wobei das erste Gehäuse weiter den ersten und zweiten optischen Steckverbinder aufnimmt und/oder wobei der erste optische Steckverbinder und/oder der zweite optische Steckverbinder außerhalb des ersten Gehäuses angeordnet und über ein flexibles optische Faserkabel, das einen Abschnitt der ersten optischen Faser aufnimmt, mit dem ersten Gehäuse und/oder die zweite optische Faser verbunden ist/sind.

**11.** Einweg-Faseroptikvorrichtung nach einem der vorstehenden Ansprüche, wobei die erste optische Faser ein proximales Ende und ein distales Ende aufweist, wobei der erste optische Steckverbinder konfiguriert ist, das proximale Ende der ersten optischen Faser optisch mit einer Endfläche einer optischen Faser der medizinischen Behandlungsvorrichtung zu koppeln, und wobei der erste optische Steckverbinder konfiguriert ist, das proximale Ende der ersten optischen Faser lösbar mit der medizinischen Behandlungsvorrichtung zu verbinden, wobei das proximale Ende der ersten optischen Faser an der Endfläche der optische Faser der medizinischen Behandlungsvorrichtung anliegt.

**12.** Einweg-Faseroptikvorrichtung nach einem der vorstehenden Ansprüche, wobei der erste optische Steckverbinder konfiguriert ist, eine Einmoden-Laserstrahlung einzukoppeln, die eine V-Zahl kleiner als 10 aufweist, beispielsweise kleiner als 3.

**13.** Einweg-Faseroptikvorrichtung nach einem der vorstehenden Ansprüche, wobei die zweite optische Faser ein proximales Ende und ein distales Ende aufweist, wobei der zweite optische Steckverbinder konfiguriert ist, das proximale Ende der zweiten optischen Faser optisch mit einer optischen Ausgangsfaser der medizinischen Behandlungsvorrichtung zu koppeln, und wobei der zweite optische Steckverbinder konfiguriert ist, das proximale Ende der zweiten optischen Faser lösbar mit der medizinischen Behandlungsvorrichtung zu verbinden, wobei sich das proximale Ende der zweiten optischen Faser in einem Abstand von einer Endfläche der optischen Ausgangsfaser der medizinischen Behandlungsvorrichtung befindet.

**14.** Einweg-Faseroptikvorrichtung nach einem der vorstehenden Ansprüche, wobei der zweite optische Steckverbinder konfiguriert ist, eine Multimode-Laserstrahlung, die eine V-Zahl größer als 10 und eine Leistung von 10 W oder mehr aufweist, in die zweite optische Faser einzukoppeln.

**15.** Faseroptische medizinische Behandlungseinrichtung, umfassend eine medizinische Behandlungsvorrichtung und eine Einweg-Faseroptikvorrichtung nach einem der vorstehenden Ansprüche, wobei die Einweg-Faseroptikvorrichtung konfiguriert ist, lösbar und optisch mit der medizinischen Behandlungsvorrichtung gekoppelt zu werden.

## Revendications

**1.** Dispositif à fibre optique jetable, dans lequel le dispositif à fibre optique jetable comprend :

- une fibre optique à double gaine au moins (1200) présentant une extrémité proximale et une extrémité distale, l'extrémité distale étant configurée pour être introduite dans le canal de travail d'un endoscope, dans lequel la fibre optique à au moins double gaine présente au moins un cœur et au moins deux gaines ; et
- un module de connexion (1100) destiné à connecter optiquement l'extrémité proximale d'une fibre optique à au moins double gaine à un dispositif de traitement médical, dans lequel le module de connexion comprend :

- une première fibre optique (1110),
- une seconde fibre optique (1120),
- un premier connecteur optique (1111) pour connecter de manière détachable et optique la première fibre optique au dispositif de traitement médical,
- un second connecteur optique (1121) pour connecter de manière amovible et optique la seconde fibre optique au dispositif de traitement médical, et
- un module combinateur optique (1130) configuré pour coupler le rayonnement entre la première fibre optique et le au moins un cœur de la fibre optique une fibre optique à au moins double gaine et pour coupler le rayonnement entre la seconde fibre optique et au moins une gaine des au moins deux gaines de la fibre optique à au moins double gaine.

**2.** Dispositif à fibre optique jetable selon la revendication 1, dans lequel le premier connecteur optique est un connecteur optique monomode à faible réflectivité assurant un contact physique entre les extrémités respectives des fibres optiques connectées optiquement par le premier connecteur optique, et dans lequel le second connecteur optique est un connecteur d'extrémité de fibre autoportant pour une connexion optique multimode haute puissance.

**3.** Dispositif à fibre optique jetable selon l'une quelconque des revendications précédentes, dans lequel le premier et/ou le second connecteur optique est à ressort.

**4.** Dispositif à fibre optique jetable selon l'une quelconque des revendications précédentes, dans lequel le module de combinaison optique inclut un combineur latéral configuré pour coupler latéralement le rayonnement entre la seconde fibre optique, ou une section de fibre multimode couplée optiquement à la seconde fibre optique, et la au moins une gaine de la fibre optique à au moins double gaine, et dans lequel le au moins un cœur de la fibre optique à au moins double gaine passe à travers le combineur latéral.

5. Dispositif à fibre optique jetable selon l'une quelconque des revendications précédentes, dans lequel le module de combinaison optique inclut un combineur latéral configuré pour coupler latéralement le rayonnement entre une section de fibre multimode présentant un premier diamètre de fibre et au moins une gaine d'une fibre optique à au moins double gaine, dans lequel au moins un cœur de la fibre optique à au moins double gaine passe à travers le combineur latéral et dans lequel la fibre optique à double gaine présente un diamètre supérieur au diamètre de la section de fibre multimode.

6. Dispositif à fibre optique jetable selon l'une quelconque des revendications précédentes, dans lequel le module de connexion comprend un capteur de température et/ou un élément de refroidissement configuré pour refroidir le module de combinaison optique.

7. Dispositif à fibre optique jetable selon l'une quelconque des revendications précédentes, dans lequel la fibre optique à au moins double gaine est une fibre multicœur présentant deux cœurs ou plus, et dans lequel la fibre optique à au moins double gaine comprend une région conique configurée pour le couplage du rayonnement entre un premier cœur des deux ou plusieurs cœurs et au moins un second cœur des deux ou plusieurs cœurs, différent du premier cœur.

8. Dispositif à fibre optique jetable selon l'une quelconque des revendications précédentes, dans lequel l'extrémité distale de la fibre optique à au moins double gaine présente une pointe angulaire.

9. Dispositif à fibre optique jetable selon l'une quelconque des revendications précédentes, dans lequel le module de connexion comprend un premier boîtier contenant au moins le module de combinaison optique et au moins une partie de la première fibre optique et au moins une partie de la seconde fibre optique.

10. Dispositif à fibre optique jetable selon la revendication 9, dans lequel le premier boîtier accueille en outre les premier et second connecteurs optiques, et/ou dans lequel le premier connecteur optique et/ou le second connecteur optique est/sont agencé(s) à l'extérieur du premier boîtier et relié(s) au premier boîtier par un câble à fibres optiques flexible accueillant une partie de la première fibre optique et/ou de la seconde fibre optique.

11. Dispositif à fibre optique jetable selon l'une quelconque des revendications précédentes, dans lequel la première fibre optique présente une extrémité proximale et une extrémité distale, dans lequel le premier connecteur optique est configuré pour coupler optiquement l'extrémité proximale de la première fibre optique à une face d'extrémité d'une fibre optique du dispositif de traitement médical, et dans lequel le premier connecteur optique est configuré pour connecter de manière amovible l'extrémité proximale de la première fibre optique au dispositif de traitement médical, l'extrémité proximale de la première fibre optique étant butant contre la face d'extrémité de la fibre optique du dispositif de traitement médical.

12. Dispositif à fibre optique jetable selon l'une quelconque des revendications précédentes, dans lequel le premier connecteur optique est configuré pour coupler un rayonnement laser monomode présentant un nombre V inférieur à 10, tel que inférieur à 3.

13. Dispositif à fibre optique jetable selon l'une quelconque des revendications précédentes, dans lequel la seconde fibre optique présente une extrémité proximale et une extrémité distale, dans lequel le second connecteur optique est configuré pour coupler optiquement l'extrémité proximale de la seconde fibre optique à une fibre optique de sortie du dispositif de traitement médical, et dans lequel le second connecteur optique est configuré pour connecter de manière amovible l'extrémité proximale de la seconde fibre optique au dispositif de traitement médical, l'extrémité proximale de la seconde fibre optique étant positionnée à une certaine distance d'une face d'extrémité de la fibre optique de sortie du dispositif de traitement médical.

14. Dispositif à fibre optique jetable selon l'une quelconque des revendications précédentes, dans lequel le second connecteur optique est configuré pour coupler un rayonnement laser multimode présentant un nombre V supérieur à 10 et une puissance de 10 W ou plus dans la seconde fibre optique.

15. Appareil de traitement médical à fibres optiques comprenant un dispositif de traitement médical et un dispositif à fibre optique jetable selon l'une quelconque des revendications précédentes, dans lequel le dispositif à fibre optique jetable est configuré pour être détaché et couplé optiquement au dispositif de traitement médical.

FIG. 1

FIG. 2

FIG. 3

*FIG. 4*

*FIG. 5*

*FIG. 6*

*FIG. 7*

1213
1211
1200
1214

FIG. 8A

1213
1200
1211
1214

FIG. 8B

1211
1200
1213
1212
1214

FIG. 8C

1211
1200
1213
1214
1212
1215

FIG. 8D

1216
1213
1211
1212
1200
1214
1215

FIG. 8E

1200
1213
1211
1217
1218
1214

FIG. 8F

1200
1201
1213

FIG. 9A

1200
1201
1213

FIG. 9B

1201
1213
1213
1200

FIG. 9C

a

FIG. 10

*FIG. 11*

FIG. 12

FIG. 13

FIG. 14A

FIG. 14B

FIG. 15A

FIG. 15B

**REFERENCES CITED IN THE DESCRIPTION**

**Patent documents cited in the description**

- US 2007282403 A1
- WO 2020112864 A1
- US 20100228119 A